# EUROPEAN PATENT APPLICATION

(11) **EP 4 495 129 A1**
(43) Date of publication of application: **22.01.2025**
(21) Application number: 23770728.6
(22) Date of filing: 13.03.2023
(51) Int. Cl.: C07H 21/04

(54) **METHOD FOR PRODUCING CHIMERIC MOLECULE**

(30) Priority: 14.03.2022 JP 2022039293
(71) Applicant: Tohoku University, Sendai-shi, Miyagi 980-8577 (JP); National University Corporation Tokai National Higher Education and Research System, Nagoya-shi, Aichi 464-8601 (JP)
(72) Inventor: WADA, Takehiko, Sendai-shi, Miyagi 980-8577 (JP); INAGAKI, Masahito, Nagoya-shi, Aichi 464-8601 (JP)
(74) Representative: Arnold & Siedsma
(86) International application number: PCT/JP2023/009612
(87) International publication number: WO 2023/176773

(57) **Abstract**

Provided is a method for producing a chimeric molecule by a liquid-phase synthesis method, including bonding an anionic main chain nucleic acid containing a DNA and a neutral or cationic main chain nucleic acid containing a PNA. A method for producing a chimeric molecule according to one aspect of the present invention is a method for producing a chimeric molecule in which a first nucleic acid and a second nucleic acid are fused. The method includes preparing a second chimeric molecule precursor through introduction of the second nucleic acid into a first chimeric molecule precursor via a hydroxyl group of the first chimeric molecule precursor, deprotecting the second chimeric molecule precursor as necessary, and introducing the first nucleic acid into the second chimeric molecule precursor. The first chimeric molecule precursor contains a lipid-soluble anchor. The second nucleic acid has an optionally protected amino group. In the deprotecting of the second chimeric molecule precursor, a protected amino group of the second nucleic acid introduced into the second chimeric molecule precursor is deprotected. In the introducing of the first nucleic acid, the first nucleic acid is introduced into the second nucleic acid via the amino group of the second nucleic acid. Each step is performed by a liquid-phase synthesis method.

## Description

### Technical Field

The present invention relates to a method for producing a chimeric molecule.

The present application claims priority on the basis of Japanese Patent Application No. 2022-039293 filed in Japan on March 14, 2022, the content of which is incorporated herein by reference.

### Background Art

In recent years, oligonucleotide therapeutics have attracted attention as next-generation molecular target drugs as well as antibody drugs.

As a pharmaceutical strategy in oligonucleotide therapeutics, for example, antisense oligonucleotides (ASO) have been known. An ASO targets a messenger RNA (mRNA), a micro RNA (miRNA), an siRNA, or the like involved in disease progression, recognizes the target in a base sequence-selective manner, and forms a complex, to suppress the function of the target RNA and exhibit a therapeutic effect. In order for such oligonucleotide therapeutics to effectively express their therapeutic activities, 1) high in vivo stability and 2) high specificity to target nucleic acids and complex stability are required, and the development of modified oligonucleic acids/artificial oligonucleic acids in which natural DNA/RNA is chemically modified has been actively studied.

As a methodology for overcoming the off-target effects in a broad sense in oligonucleotide therapeutics (inherent toxicity of oligonucleotide therapeutics independent of target nucleic acid recognition), reduction of the dose of oligonucleotide therapeutics has been proposed. However, when the dose is reduced, the amount of the complex formed with the target RNA naturally decreases, and effective therapeutic activities expression cannot be expected. As a solution to this issue, a nucleic acid drug having a catalyst-like function utilizing RNaseH, which cleaves a target RNA like a catalyst with a small amount of an ASO, has attracted attention (Non-Patent Document 1).

As a nucleic acid drug having a catalyst-like function utilizing RNaseH, there has been reported a chimeric molecule that is useful for constructing an oligonucleic acid system capable of rapidly dissociating from a complex of a target RNA after cleavage, focusing on a dissociation process after RNA cleavage, and capable of inhibiting the function of the target nucleic acid at a low concentration and suppressing off-target effects (Patent Document 1).

The solid-phase synthesis method is widely used for synthesis of oligonucleic acids. The solid-phase synthesis method is also widely used for peptide synthesis.

Although the solid-phase synthesis method has been optimized in process and automated, it is essentially heterogeneous, and in order to compensate for its low reactivity, it is necessary to use a large excess amount of a reagent, for example, 6 to 10 equivalents or more, and there is room for improvement in terms of cost. It must be said that the solid-phase synthesis method is difficult to apply to industrialization although it is applicable at laboratory scale, because the method needs advanced techniques for isolation and purification and is difficult to be scaled up due to the limit of the amount of functional groups supported on a solid-phase resin for reaction.

As an attempt to solve the aforementioned drawbacks of the solid-phase synthesis method while taking advantage of the solid-phase synthesis method in which isolation and purification after the reaction can be performed only by filtration and washing, methods have been proposed in which only a specific component dissolved in a liquid phase is precipitated and isolated as a solid to facilitate isolation and purification after the reaction (Patent Documents 2 to 7).

### Citation List

### Patent Document

Patent Document 1: WO 2021/015234 A
Patent Document 2: WO 2012/157723 A
Patent Document 3: WO 2014/189142 A
Patent Document 4: WO 2010/104169 A
Patent Document 5: WO 2010/113939 A
Patent Document 6: WO 2011/078295 A
Patent Document 7: WO 2016/117663 A

### Non-Patent Document

Non-Patent Document 1: Liang, X. et al., Mol. Ther., 2017, 25(9), 2075

### Summary of the Invention

### Technical Problem

For example, as described in aforementioned Patent Documents 2 to 7, there have been reports on production of oligomers without fusion of different kinds of molecules, such as oligoribonucleic acid, oligodeoxyribonucleic acid, or oligopeptide, by a liquid-phase synthesis method.

However, as a method for producing a chimeric molecule in which, as a typical example, a ribonucleic acid (RNA) or a deoxyribonucleic acid (DNA) and a peptide nucleic acid (PNA) or a peptide ribonucleic acid (PRNA) having an oligopeptide skeleton are fused in any combination as reported in Patent Document 1, only a production method by a solid-phase synthesis method is known.

In particular, there are no reports of liquid-phase synthesis methods relating to the introduction of a PNA or a PRNA into an oligo RNA or an oligo DNA via the terminal nitrogen atom of the oligo RNA or the oligo DNA or the introduction of an RNA or a DNA into an oligo PNA or an oligo PRNA via the terminal nitrogen atom of the oligo PNA or the oligo PRNA.

That is, although a liquid-phase synthesis method has been reported as a technique for binding molecules of the same kind, at least a part of the steps of constructing a PNA-DNA structure in which a PNA or a PRNA is fused to an RNA or a DNA, or a DNA-PNA structure in which an RNA or a DNA is fused to a PNA or a PRNA must be performed by a solid-phase synthesis at present.

In the solid-phase synthesis method, the technique of further elongating a DNA using the DNA as a starting point allows the reaction to proceed almost quantitatively in each step using an automatic synthesizer, and thus a sufficient yield can be maintained even when a plurality of DNAs is elongated. On the other hand, when a PNA-DNA structure is constructed using a DNA as a starting point in the solid-phase synthesis method, the yield is only about 70% at the maximum, and the yield decreases by several percent as the elongation of the PNA further proceeds. As a result, even when the solid-phase synthesis method is used, the construction of the PNA-DNA structure does not give a satisfactory yield, the reaction system becomes complicated because of many side reactions, and a great deal of labor is required for isolation.

In view of the above issues, an object of the present invention is to provide a method for producing a chimeric molecule by a liquid-phase synthesis method, including production of a second chimeric molecule precursor described below and production of a chimeric molecule using the second chimeric molecule precursor, in which a nucleic acid having a neutral or cationic backbone represented by a PNA or a PRNA or a derivative thereof is introduced into a nucleic acid having an anionic backbone represented by an RNA or a DNA or a derivative thereof.

An object of another aspect of the present invention is to provide a method for producing a chimeric molecule by a liquid-phase synthesis method, including production of a chimeric molecule using a third chimeric molecule precursor described below, in which a nucleic acid having an anionic backbone represented by an RNA or a DNA or a derivative thereof is introduced into a nucleic acid having a neutral or cationic backbone represented by a PNA or a PRNA or a derivative thereof.

### Solution to Problem

The present invention has the following gist.

A method for producing a chimeric molecule in which at least one first nucleic acid having a neutral or cationic backbone or a derivative thereof and at least one second nucleic acid having an anionic backbone or a derivative thereof are fused, the method including:
preparing a second chimeric molecule precursor through introduction of the second nucleic acid or a derivative thereof into a first chimeric molecule precursor having a hydroxyl group via the hydroxyl group of the first chimeric molecule precursor having a hydroxyl group;
deprotecting the second chimeric molecule precursor as necessary; and
introducing the first nucleic acid or a derivative thereof into the second chimeric molecule precursor or the deprotected second chimeric molecule precursor,
wherein
the first chimeric molecule precursor having a hydroxyl group contains a lipid-soluble anchor,
the second nucleic acid or a derivative thereof has an optionally protected amino group,
in the deprotecting of the second chimeric molecule precursor, a protected amino group of the second nucleic acid or a derivative thereof introduced into the second chimeric molecule precursor is deprotected,
in the introducing of the first nucleic acid or a derivative thereof, the first nucleic acid or a derivative thereof is introduced into the second nucleic acid or a derivative thereof via the amino group of the second nucleic acid or a derivative thereof, and
each of the preparing of the second chimeric molecule precursor, the deprotecting of the second chimeric molecule precursor, and the producing of the chimeric molecule is performed by a liquid-phase synthesis method.

Preferably, the first nucleic acid is a PNA, a PRNA, a PNA/PRNA, or an LNA, and the second nucleic acid is an RNA or a DNA.

Preferably, the first nucleic acid is a PNA and the second nucleic acid is a DNA.

Preferably, the chimeric molecule includes a portion at which the first nucleic acid or a derivative thereof is bound at a 5'-terminal of the second nucleic acid or a derivative thereof.

The first chimeric molecule precursor is preferably a compound represented by a specific formula described below.

The second nucleic acid or a derivative thereof is preferably a compound represented by a specific formula described below.

Preferably, the optionally protected amino group is a protected amino group, and a protective group of the protected amino group is an optionally substituted trityl group.

The first nucleic acid or a derivative thereof is preferably a compound represented by a specific formula described below.

Another aspect of the present invention has the following gist.

A method for producing a chimeric molecule in which at least one first nucleic acid having a neutral or cationic backbone or a derivative thereof and at least one second nucleic acid having an anionic backbone or a derivative thereof are fused,
the method including:
introducing the second nucleic acid or a derivative thereof into a third chimeric molecule precursor having an amino group via the amino group of the third chimeric molecule precursor having an amino group,
wherein
the third chimeric molecule precursor having an amino group includes a partial structure derived from the first nucleic acid or a derivative thereof, an amino group derived from the first nucleic acid or a derivative thereof, and a lipid-soluble anchor, and
the introducing of the second nucleic acid or a derivative thereof into the third chimeric molecule precursor having an amino group is performed by a liquid-phase synthesis method.

Preferably, the first nucleic acid is a PNA, a PRNA, a PNA/PRNA, or an LNA, and the second nucleic acid is an RNA or a DNA.

Preferably, the first nucleic acid is a PNA and the second nucleic acid is a DNA.

Preferably, the chimeric molecule includes a portion at which the first nucleic acid or a derivative thereof is bound at a 3'-terminal of the second nucleic acid or a derivative thereof.

The third chimeric molecule precursor is preferably a compound represented by a specific formula described below.

The second nucleic acid or a derivative thereof is preferably a compound represented by a specific formula described below.

Still another aspect of the present invention has the following gist.
[1] A method for producing a chimeric molecule in which at least one first nucleic acid that is a PNA, a PRNA, a PNA/PRNA, or an LNA, or a derivative thereof, and at least one second nucleic acid that is an RNA or a DNA, or a derivative thereof are fused,
   the derivative of the first nucleic acid being a halogenated derivative of a base moiety bound to the first nucleic acid, a deaminated derivative of a base moiety bound to the first nucleic acid, or a derivative of a base moiety bound to the first nucleic acid having a sulfur atom instead of an oxygen atom,
   the derivative of the second nucleic acid being a halogenated derivative of a base moiety bound to the second nucleic acid, a deaminated derivative of a base moiety bound to the second nucleic acid, a derivative of a base moiety bound to the second nucleic acid having a sulfur atom instead of an oxygen atom, a phosphorothioate type DNA or RNA, a phosphorodithioate type DNA or RNA, or a morpholino type nucleic acid,
   the base moiety meaning uracil, cytosine, thymine, adenine, guanine, a purine ring, or a pyrimidine ring bound to a nucleic acid,
   the method including:
      preparing a second chimeric molecule precursor through introduction of the second nucleic acid or a derivative thereof into the first chimeric molecule precursor having a hydroxyl group via a hydroxyl group at a 5'- position of the RNA or DNA of the first chimeric molecule precursor having a hydroxyl group;
      oxidizing a phosphorus atom of the second chimeric molecule precursor with a peroxide;
      deprotecting the second chimeric molecule precursor as necessary; and
      introducing the first nucleic acid or a derivative thereof into the second chimeric molecule precursor or the deprotected second chimeric molecule precursor,
      wherein
      the first chimeric molecule precursor has a structure represented by Formula (1) or (2):

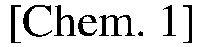

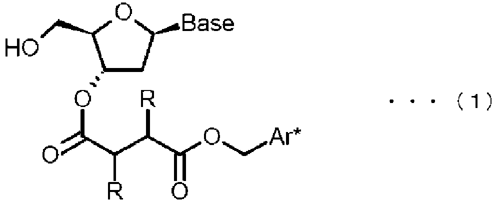

         where
         Ar* represents an aromatic hydrocarbon ring having 6 to 14 carbon atoms to which an aliphatic hydrocarbon group having 10 to 40 carbon atoms is bound via a single bond or a linker,
         R's each independently represent a hydrogen atom or an alkyl group having 1 to 6 carbon atoms, Base represents the base moiety, and
         deoxyribose may have an optionally protected hydroxyl group at a 2'-position, and the linker is -O-, -C(=O)-, -C(=O)-O-, -O-C(=O)-, -C(=O)-NH-, -NH-C(=O)-, -S-, -S(=O)-, or - S(=O)₂-; 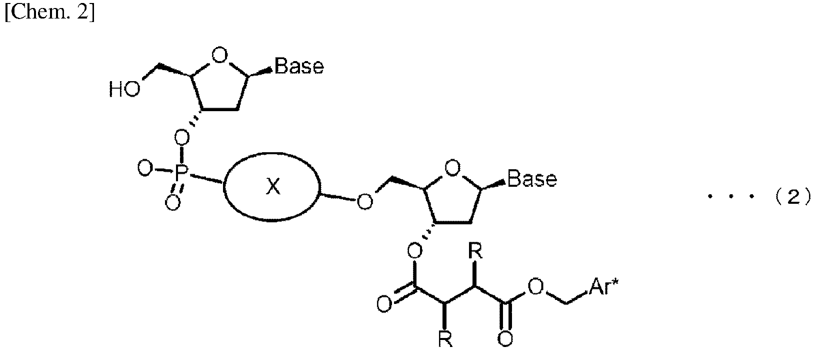 where
            Ar* represents an aromatic hydrocarbon ring having 6 to 14 carbon atoms to which an aliphatic hydrocarbon group having 10 to 40 carbon atoms is bound via a single bond or a linker,
            R's each independently represent a hydrogen atom or an alkyl group having 1 to 6 carbon atoms,
            Base represents the base moiety,
            X represents a single bond, or represents one or more first nucleic acids or derivatives thereof and/or second nucleic acids or derivatives thereof through which an oxygen atom bound to X and a phosphorus atom bound to X are held together,
            deoxyribose may have an optionally protected hydroxyl group at a 2'-position, and the linker is -O-, -C(=O)-, -C(=O)-O-, -O-C(=O)-, -C(=O)-NH-, -NH-C(=O)-, -S-, -S(=O)-, or - S(=O)₂-;
            the second nucleic acid has a structure represented by Formula (4): 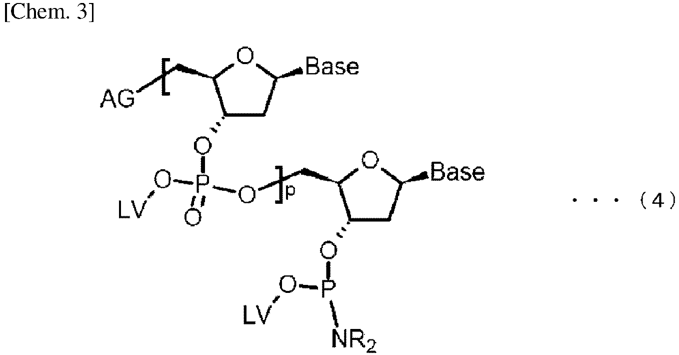 where
               p represents an integer of 0 or more,
               AG represents an optionally protected amino group,
               Base represents a base moiety,
               NR₂ represents a dialkylamino group,
               LV represents a 2-cyanoethyl group, an allyl group, or a benzyl group, and
               deoxyriboses may each independently have an optionally protected hydroxyl group at a 2'-position,
               in the deprotecting of the second chimeric molecule precursor, the protected amino group of the second nucleic acid or a derivative thereof introduced into the second chimeric molecule precursor is deprotected,
               in the introducing of the first nucleic acid or a derivative thereof, the first nucleic acid or a derivative thereof is introduced into the second nucleic acid or a derivative thereof via the amino group of the second nucleic acid or a derivative thereof,
               the first nucleic acid has a structure represented by Formula (5): 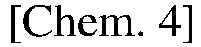 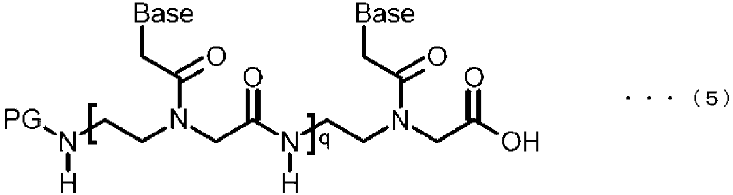 where
                  q represents an integer of 0 or more,
                  PG represents a protective group of an adjacent amino group, and
                  Base represents the base moiety; and
                  each of the preparing of the second chimeric molecule precursor, the deprotecting of the second chimeric molecule precursor, and the producing of the chimeric molecule is performed by a liquid-phase synthesis method.
[2] The method according to [1], wherein the first nucleic acid is a PNA and the second nucleic acid is a DNA.
[3] The method according to [1] or [2], wherein the first nucleic acid or a derivative thereof is a PNA, and the second nucleic acid or a derivative thereof is a DNA.
[4] The method according to any of [1] to [3], wherein the peroxide is tert-butyl hydroperoxide or meta-chloroperbenzoic acid, and a use amount of the peroxide to be used is from 2 to 5 moles per number of moles of the second chimeric molecule precursor used or from 2 to 5 moles per number of moles of the first chimeric molecule precursor used for preparing the second chimeric molecule precursor.
[5] The method according to any of [1] to [4], wherein the linker in Formula (1) and the linker in Formula (2) are -O-, the aromatic hydrocarbon ring having 6 to 14 carbon atoms is a benzene ring, and R is a hydrogen atom.
[6] The method according to any of [1] to [5], wherein LV in Formula (4) is a 2-cyanoethyl group.
[7] The method according to any of [1] to [6], wherein in AG of Formula (4), the optionally protected amino group is a protected amino group, and a protective group of the protected amino group is a trityl group, a p-methoxyphenyldiphenylmethyl group, or a di(p-methoxyphenyl)phenylmethyl group.
[8] The method according to any of [1] to [7], wherein, in PG of Formula (5), the protective group for an adjacent amino group is a fluorenylmethoxycarbonyl group in which a fluorenyl group may be substituted with a group selected from the group consisting of halogen, nitro, cyano, trifluoromethyl, carboxy, alkyloxycarbonyl, dialkylcarbamoyl, and alkylcarbonyl; a trityl group; a p-methoxyphenyldiphenylmethyl group; a di(p-methoxyphenyl) phenylmethyl group; or a phthalimide-type protecting group.
[9] The method according to [1], wherein the optionally protected hydroxyl group allowed to be substituted at the 2'-position of the deoxyribose in Formula (1), Formula (2), or Formula (4) is an unprotected hydroxyl group.
[10] A method for producing a chimeric molecule in which at least one first nucleic acid that is a PNA, a PRNA, a PNA/PRNA, or an LNA, or a derivative thereof, and at least one second nucleic acid that is an RNA or a DNA, or a derivative thereof are fused,
   the derivative of the first nucleic acid being a halogenated derivative of a base moiety bound to the first nucleic acid, a deaminated derivative of a base moiety bound to the first nucleic acid, or a derivative of a base moiety bound to the first nucleic acid having a sulfur atom instead of an oxygen atom,
   the derivative of the second nucleic acid being a halogenated derivative of a base moiety bound to the second nucleic acid, a deaminated derivative of a base moiety bound to the second nucleic acid, a derivative of a base moiety bound to the second nucleic acid having a sulfur atom instead of an oxygen atom, a phosphorothioate type DNA or RNA, a phosphorodithioate type DNA or RNA, or a morpholino type nucleic acid,
   the base moiety meaning uracil, cytosine, thymine, adenine, guanine, a purine ring, or a pyrimidine ring bound to a nucleic acid,
   the method including
   introducing the second nucleic acid or a derivative thereof into a third chimeric molecule precursor having an amino group via the amino group of the third chimeric molecule precursor having an amino group,
   wherein
   the third chimeric molecule precursor has a structure represented by Formula (11) or (12):

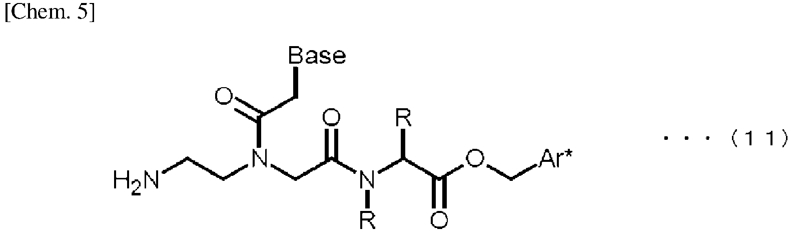

      where
      Ar* represents an aromatic hydrocarbon ring having 6 to 14 carbon atoms to which an aliphatic hydrocarbon group having 10 to 40 carbon atoms is bound via a single bond or a linker,
      R's each independently represent a hydrogen atom or an alkyl group having 1 to 6 carbon atoms, and
      Base represents the base moiety, and the linker is -O-, -C(=O)-, -C(=O)-O-, -O-C(=O)-, - C(=O)-NH-, -NH-C(=O)-, -S-, -S(=O)-, or -S(=O)₂-; 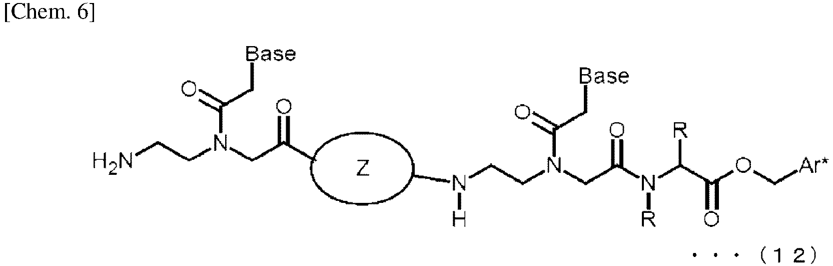 where
         Ar* represents an aromatic hydrocarbon ring having 6 to 14 carbon atoms to which an aliphatic hydrocarbon group having 10 to 40 carbon atoms is bound via a single bond or a linker,
         R's each independently represent a hydrogen atom or an alkyl group having 1 to 6 carbon atoms,
         Base represents the base moiety,
         Z represents a single bond, or represents one or more first nucleic acids or a derivative thereof and/or second nucleic acids or a derivative thereof through which a nitrogen atom bound to Z and a carbonyl carbon atom bound to Z are held together, and
         deoxyriboses may each have an optionally protected hydroxyl group at a 2'-position, and the linker is -O-, -C(=O)-, -C(=O)-O-, -O-C(=O)-, -C(=O)-NH-, -NH-C(=O)-, -S-, -S(=O)-, or - S(=O)₂-;
         the second nucleic acid has a structure represented by Formula (14):

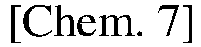

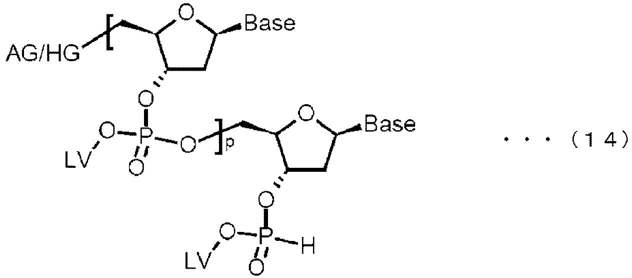

            where
            p represents an integer of 0 or more,
            AG/HG represents an optionally protected amino group or hydroxyl group,
            Base represents the base moiety,
            LV represents a 2-cyanoethyl group, an allyl group, or a benzyl group, and
            deoxyriboses may each independently have an optionally protected hydroxyl group at a 2'-position; and
            the introducing of the second nucleic acid or a derivative thereof into the third chimeric molecule precursor having an amino group is performed by a liquid-phase synthesis method.

### Advantageous Effects of Invention

An aspect of the present invention provides a method for producing a chimeric molecule by a liquid-phase synthesis method in which a nucleic acid having a neutral or cationic backbone represented by a PNA or a PRNA or a derivative thereof is introduced into a nucleic acid having an anionic backbone represented by an RNA or a DNA or a derivative thereof.

This method makes it possible to produce, by a liquid-phase synthesis method, a PNA-DNA structure in which a PNA structure is fused to an oligo RNA or an oligo DNA, or a PNA-DNA structure in which a PNA structure is further bound to the PNA-DNA structure; or a PNA-DNA-PNA structure in which a PNA structure is further fused to a DNA-PNA structure, or a PNA-DNA-PNA structure in which a PNA structure is further bound to the PNA-DNA-PNA structure.

Another aspect of the present invention provides a method for producing a chimeric molecule by a liquid-phase synthesis method in which a nucleic acid having an anionic backbone represented by an RNA or a DNA or a derivative thereof is introduced into a nucleic acid having a neutral or cationic backbone represented by a PNA or a PRNA or a derivative thereof.

This method makes it possible to produce, by a liquid-phase synthesis, a DNA-PNA structure in which a DNA structure or an RNA structure is fused to an oligo PNA or an oligo PRNA, or a DNA-PNA structure in which a DNA structure or an RNA structure is further bound to the DNA-PNA structure; or a DNA-PNA-DNA structure in which a DNA structure or an RNA structure is fused to a PNA-DNA structure, or a DNA-PNA-DNA structure in which a DNA structure or an RNA structure is further bound to the DNA-PNA-DNA structure.

### Description of Embodiment

Hereinafter, embodiments of the present invention will be specifically described. The present invention is not limited to the following embodiments, and various modifications can be made within the scope of the gist thereof.

The chimeric molecule that is the objective substance of the production method of the present invention is a compound in which at least one first nucleic acid having a neutral or cationic backbone or a derivative thereof (hereinafter, also simply referred to as "first nucleic acid" including the derivative) and at least one second nucleic acid having an anionic backbone or a derivative thereof (hereinafter, also simply referred to as "second nucleic acid" including the derivative) are fused. That is, the chimeric molecule that is the objective substance of the production method of the present invention only needs to have a partial structure in which the first nucleic acid and the second nucleic acid are fused.

In the chimeric molecule that is the objective substance of the production method of the present invention, a plurality of nucleic acids is bound, any part thereof may be the first nucleic acid, and the remaining part may be the second nucleic acid.

In the chimeric molecule in which a plurality of nucleic acids is bound, the lower limit of the total number of nucleic acids may be 2, 3, 5, 10, or 16. The upper limit of the total number of nucleic acids may be 80, 40, 30, 23, 15, 10, or 5. Any one of the above-described upper limits and any one of the above-described lower limits can be combined in any combination. For example, the total number may be from 2 to 80, from 2 to 40, from 2 to 15, from 2 to 10, from 2 to 5, from 3 to 80, from 3 to 40, from 3 to 10, from 5 to 40, from 10 to 30, or from 16 to 23.

In the chimeric molecule in which a plurality of nucleic acids is bound, the ratio pf the number of the first nucleic acids to the number of the second nucleic acids in the total number of the nucleic acids is not particularly limited. The ratio of the number of the first nucleic acids to the number of the second nucleic acids may be 1:10 to 10:1, preferably 1:3 to 3:1, and more preferably 1:2 to 2:1.

Each of the first nucleic acid and the second nucleic acid in the present invention preferably has an ability to bind to a target nucleic acid.

The target nucleic acid is not particularly limited as long as it is a nucleic acid having a target sequence to which the chimeric molecule that is the objective substance of the production method of the present invention can bind or a derivative thereof, but is preferably an RNA or a DNA. When the chimeric molecule is used as an active ingredient of a pharmaceutical composition, the target nucleic acid is preferably an RNA or a DNA encoding a protein causing a disease to be treated using the pharmaceutical composition, or an mRNA, an miRNA, or an siRNA associated with the disease.

In the present invention, the nucleic acid means a nucleic acid in a broad sense including RNAs and DNAs which are generally defined as nucleic acids, and PNAs, PRNAs, LNAs (bridged artificial nucleic acid) and the like which are so-called artificial nucleic acids. The derivative of the nucleic acid is not particularly limited, and examples thereof include a halogenated derivative and a deaminated derivative of a base moiety (uracil, cytosine, thymine, adenine, guanine, or a purine ring or a pyrimidine ring) bound to the nucleic acid, and a derivative having a sulfur atom instead of an oxygen atom of each nucleic acid base. Examples of the derivative of the nucleic acid include, in addition to phosphorothioate type DNAs and RNAs and phosphorodithioate type DNAs and RNAs, morpholino type nucleic acids as disclosed in aforementioned Patent Document 3.

In the present specification, "halogen" means fluoro, chloro, bromo, or iodo, preferably fluoro, chloro, or bromo.

The ribose or deoxyribose in an RNA, a DNA, or a PRNA may be a ribose or deoxyribose in which the carbon atoms at the 2- and 4-positions of an LNA or the like are bound by a divalent organic group, and examples of such ribose or deoxyribose include a ribose or deoxyribose having the structure described in aforementioned Patent Document 7. The ribose or deoxyribose in an RNA, a DNA, or a PRNA is preferably a ribose or deoxyribose.

In the present invention, the first nucleic acid having a neutral or cationic backbone or a derivative thereof preferably has a neutral backbone.

The neutral backbone is not particularly limited, and examples thereof include an amide skeleton (typically, a skeleton having N-(2-aminoethyl) glycine as a unit). Examples of the nucleic acid having an amide skeleton or a derivative thereof include a PNA or a derivative thereof, a PRNA or a derivative thereof, and a combination of a PNA and a PRNA (hereinafter also referred to as "PNA/PRNA") or a derivative thereof.

In the PNA/PRNA, the bonding position of the PRNA in the PNA may be any position of the PNA, and the PRNA may be bound in the middle of the PNA. For example, it may be a combination such as PNA-PRNA-PNA.

The cationic backbone is not particularly limited, and examples thereof include an imino skeleton, a phosphoric acid amide skeleton, a phosphoramidite skeleton, and a skeleton having a cationic side chain such as an amino group or a guanidium group in a side chain of an amide skeleton side chain.

In the second nucleic acid or a derivative thereof having an anionic backbone in the present invention, the anionic backbone is not particularly limited, and examples thereof include a sugar-phosphate skeleton and a sugar-thiophosphate skeleton. Examples of the nucleic acid having a sugar-phosphate skeleton or a sugar-thiophosphate skeleton or a derivative thereof include an RNA or a derivative thereof, and a DNA or a derivative thereof.

In the chimeric molecule, the first nucleic acid or a derivative thereof is preferably any one selected from the group consisting of PNAs, PRNAs, PNA/PRNAs, LNAs, and derivatives thereof, and more preferably any one selected from the group consisting of PNAs and derivatives thereof. In the chimeric molecule, the second nucleic acid or a derivative thereof is preferably any one selected from the group consisting of RNAs, DNAs, and derivatives thereof. In particular, in the chimeric molecule, it is preferable that the first nucleic acid or derivative thereof is selected from the group consisting of PNAs, PRNAs, PNA/PRNAs, LNAs, and derivatives thereof, and the second nucleic acid or derivative thereof is selected from the group consisting of RNAs, DNAs, and derivatives thereof; it is more preferable that the first nucleic acid or derivative thereof is selected from the group consisting of PNAs and derivatives thereof, and the second nucleic acid or derivative thereof is selected from the group consisting of RNAs, DNAs, and derivatives thereof; it is still more preferable that the first nucleic acid or derivative thereof is selected from the group consisting of PNAs and derivatives thereof, and the second nucleic acid or derivative thereof is selected from the group consisting of DNAs and derivatives thereof. When a plurality of first nucleic acids or derivatives thereof or a plurality of second nucleic acids or derivatives thereof is contained in the chimeric molecule, the plurality of first nucleic acids or derivatives thereof may be the same or different from each other, and the plurality of second nucleic acids or derivatives thereof may be the same or different from each other.

In the chimeric molecule, the first nucleic acid may be bound to either the 3' terminal or the 5' terminal of the second nucleic acid.

In one aspect of the present invention, it is preferable that there is at least one portion where the first nucleic acid is bound at the 5' terminal of the second nucleic acid. Alternatively, it is necessary that there is at least one portion where the first nucleic acid is bound at the 5' terminal of the second nucleic acid.

In another aspect of the present invention, it is preferable that there is at least one portion where the first nucleic acid is bound at the 3' terminal of the second nucleic acid. Alternatively, it is necessary that there is at least one portion where the first nucleic acid is bound at the 3' terminal of the second nucleic acid.

Examples of the chimeric molecule in which at least one first nucleic acid and at least one second nucleic acid are fused include a fusant of a PNA (or a derivative thereof. The same applies hereinafter) as the first nucleic acid and a DNA as the second nucleic acid, and a fusant of a PNA/PRNA as the first nucleic acid and a DNA as the second nucleic acid.

Examples of the fusant of a PNA and a DNA include a chimeric molecule in which the PNA is fused to the 5'-position side of the DNA (hereinafter also referred to as "PNA-DNA chimeric molecule") and a chimeric molecule in which the PNA is fused to the 3'-position side of the DNA (hereinafter also referred to as "DNA-PNA chimeric molecule"). In one aspect of the present invention, a PNA-DNA chimeric molecule is preferable. In another aspect of the present invention, a DNA-PNA chimeric molecule is preferable.

Examples of the fusant of a PNA/PRNA and a DNA include a chimeric molecule in which a PNA/PRNA is fused to the 5'-position side of a DNA (hereinafter also referred to as "PNA/PRNA-DNA chimeric molecule" or " P_{R}PD") and a chimeric molecule in which a PNA/PRNA is fused to the 3'-position side of a DNA (hereinafter also referred to as "DNA-PNA/PRNA chimeric molecule" or "DP_{R}P"). In one aspect of the present invention, P_{R}PD is preferable. In another aspect of the present invention, DP_{R}P is preferable.

In the chimeric molecule that is the objective substance of the production method of the present invention, a plurality of nucleic acids is bound, any part thereof may be the first nucleic acid, and the remaining part may be the second nucleic acid. Thus, a chimeric molecule in which the first nucleic acid and the second nucleic acid are combined in any order can be produced by combining
· an elongation of the first nucleic acid using the first nucleic acid as a starting point (preferably via the N-terminal of the first nucleic acid) (preferably an elongation to produce a bond between the N-terminal of the first nucleic acid and the C-terminal of the first nucleic acid)
· an elongation of the second nucleic acid using the first nucleic acid as a starting point (preferably via the N-terminal of the first nucleic acid) (preferable an elongation to produce a bond between the N-terminal of the first nucleic acid and the 3'-position of the second nucleic acid)
· an elongation of the first nucleic acid using the second nucleic acid as a starting point (preferably via the 5'-position of the second nucleic acid) (preferably an elongation to produce a bond between the 5'-position of the second nucleic acid and the C-terminal of the first nucleic acid), and
· an elongation of the second nucleic acid using the second nucleic acid as a starting point (preferably via the 5'-position of the second nucleic acid) (preferably an elongation to produce a bond between the 5'-position of the second nucleic acid and the 3'-position of the second nucleic acid).
Among them, for the elongation of the first nucleic acid using the first nucleic acid as a starting and the elongation of the second nucleic acid using the second nucleic acid as a starting point, a known method including a solid-phase synthesis method can be employed.

For example, the elongation of the first nucleic acid using the first nucleic acids as a starting point can be performed by employing the methods described in aforementioned Patent Documents 4 to 6, K. Ogami, et al., Chem. Lett., 2018, 47, 138 to 140, or methods similar to these methods.

For example, for the elongation of the second nucleic acid using the second nucleic acid as a starting point, the methods described in aforementioned Patent Documents 2 and 7 or methods similar to these methods can be employed.

For the elongation of the first nucleic acid using the second nucleic acid as a starting point, in particular, the elongation to produce a bond with the C-terminal of the first nucleic acid via the 5'-position of the second nucleic acid, a production method of the present invention described below (hereinafter also referred to as a first aspect of the present invention) can be employed.

For the elongation of the second nucleic acid using the first nucleic acid as a starting point, in particular, the elongation of the second nucleic acid to the N-terminal of the first nucleic acid via the 3'-position of the second nucleic acid, a production method of the present invention described below (hereinafter also referred to as a second aspect of the present invention) can be employed.

### [First Aspect of Present Invention]

A method for producing a chimeric molecule according to the first aspect includes steps (A1) to (A3) described below:
(A1) introducing the second nucleic acids into a first chimeric molecule precursor having a hydroxyl group via the hydroxyl group of the first chimeric molecule precursor having a hydroxyl group;
(A2) deprotecting the second chimeric molecule precursor as necessary; and
(A3) introducing the first nucleic acids into the second chimeric molecule precursor or the deprotected second chimeric molecule precursor.

Hereinafter, the production method according to the first aspect of the present invention will be described. The production of a PNA-DNA chimeric molecule (a chimeric molecule in which a PNA is fused to the 5'-position side of a DNA) using a PNA as the first nucleic acid and a DNA as the second nucleic acid as needed will be described as an example.

### (A1) Introducing of the second nucleic acid into a first chimeric molecule precursor having a hydroxyl group (hereinafter also simply referred to as "first chimeric molecule precursor") via the hydroxyl group of the first chimeric molecule precursor(preparing the second chimeric molecule precursor)

This step is a step of introducing the second nucleic acid into the first chimeric molecule precursor using a hydroxyl group of the first chimeric molecule precursor as a starting point. As a result, a second chimeric molecule precursor is produced.

In addition to having a hydroxyl group for introducing the second nucleic acid into the first chimeric molecule precursor, the first chimeric molecule precursor includes a lipid-soluble anchor that contributes to easy recovery by solidifying the objective substance with an addition of a specific solvent to the solution after the reaction.

The second nucleic acid has an optionally protected amino group at the 5'-position in order to introduce the first nucleic acid into the second chimeric molecule precursor or the deprotected second chimeric molecule precursor using the introduced second nucleic acid as a starting point after the introduction of the second nucleic acid into the first chimeric molecule precursor.

In the introduction of the second nucleic acid into the first chimeric molecule precursor in this step, the second nucleic acid is subjected to nucleophilic attack from the hydroxyl group of the first chimeric molecule precursor to form a bond between the oxygen atom derived from the hydroxyl group of the first chimeric molecule precursor and the phosphorus atom of the DNA as the second nucleic acid, preferably the phosphorus atom connected to the 3'-position of the deoxyribose of the DNA. The second nucleic acid may be one in which two or more second nucleic acids are bound.

The phosphorus atom of the second nucleic acid that undergoes nucleophilic attack is preferably trivalent. In this case, the trivalent phosphorus atom is bound to the oxygen atom at the 3'-position of deoxyribose (ribose in the case of RNA), a leaving group, and a group capable of finally generating -O⁻.

The leaving group may be any leaving group that is used in a nucleophilic substitution reaction of the oxygen atom to the phosphorus atom under a weakly acidic condition. Examples thereof include a dialkylamino group. As the dialkylamino group, a diisopropylamino group is preferably used, and for example, a diethylamino group or an ethylisopropylamino group can also be used.

The group capable of finally generating -O⁻ may be a group having an oxygen atom to which a substitution group capable of leaving from the oxygen atom under a condition not affecting other structures in the chimeric molecule or the chimeric molecule precursor is bound. Examples thereof include an ethoxy group having a group substituted with an electron withdrawing group at the 2-position, which can generate -O⁻ under a weakly basic condition, and a 2-cyanoethoxy group is preferable. Examples thereof also include an allyloxy group capable of generating -O⁻ by oxidative addition of a palladium catalyst in the presence of a palladium catalyst and a benzyloxy group capable of generating -O⁻ by catalytic hydrogen reduction in the presence of palladium-supported carbon.

A group capable of generating -O⁻ under a weak base condition, such as an ethoxy group having a group substituted with an electron withdrawing group at the 2-position, can finally be eliminated upon cleavage of the lipid-soluble anchor. The group that leaves from the oxygen atom under a weak base condition is a functional group necessary for the nucleophilic attack from the first chimeric molecule precursor in this step, but the group may or may leave in other steps, may be positively eliminated, or may be eliminated due to the reaction conditions.

Examples of the optionally protected amino group of the second nucleic acid (as described below, the first nucleic acid is introduced via this amino group) include an amino group and a protected amino group. To allow this step to proceed smoothly, a protected amino group is preferable.

The protective group for an amino group is not particularly limited as long as it is a group that can be removed under an acidic condition. Examples thereof include groups described in "Greene's Protective Groups in Organic Synthesis (4th edition, 2006)" written by P. G. M. Wuts and T. W. Greene, and preferred examples include an optionally substituted trityl group. Examples of the optionally substituted trityl group include a trityl group (Tr), a p-methoxyphenyldiphenylmethyl group (MMTr), and a di(p-methoxyphenyl) phenylmethyl group (DMTr), and more preferred examples include MMTr.

When a compound in which two or more second nucleic acids are bound is used as the second nucleic acid in this step, the optionally protected amino group of the second nucleic acid may be possessed only by the second nucleic acid present at the 5'-terminal of the compound in which two or more second nucleic acids are bound, and each of the second nucleic acids other than the second nucleic acid present at the 5'-terminal may be bound via an oxygen atom.

For example, the second nucleic acid used in this step has the following structure. 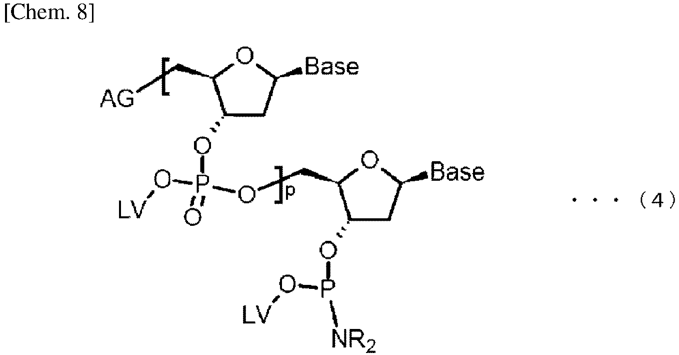
In the formula, p represents an integer of 0 or more;
AG represents an optionally protected amino group;
Base represents a base moiety (each of which may be the same or different);
NR₂ represents a leaving group used in a nucleophilic substitution reaction of an oxygen atom to a phosphorus atom under a weakly acidic condition; and
LV represents a group capable of leaving from an oxygen atom.
Deoxyriboses may each independently have an optionally protected hydroxyl group at the 2'-position.

p is preferably from 0 to 40, more preferably from 0 to 20, still more preferably from 0 to 10, yet still more preferably from 0 to 5, particularly preferably from 0 to 1, and particularly preferably 0.

Examples of the optionally protected hydroxyl group allowed to be present at the 2'-position of deoxyribose include 2'-modified compounds represented by a methoxy group or a methoxyethyloxy group, and those that can be selectively removed after synthesis to produce a hydroxyl group, such as a tert-butyldimethylsilyloxy group, triisopropyloxymethyloxy group, a tetrahydropyranyloxy group, a bis(2-acetylmethoxy)methyloxy group, and a levulinyloxy group. Hereinafter, in the present specification, the same applies to deoxyribose that is allowed to have an optionally protected hydroxyl group at the 2'-position.

The second nucleic acid used in this step, for example, a second nucleic acid in which a phosphorus atom is bonded to the oxygen atom at the 3'-position of deoxyribose, a leaving group, and a group capable of finally producing -O⁻, can be produced, for example, by the method described in US2020/0399304, Tetrahedron Letters, 39(24), 4215-4218, 1998, or Journal of Carbohydrate Chemistry, 24(2), 145-160, 2005, or a method similar to these methods.

The lipid-soluble anchor of the first chimeric molecule precursor may be a partial structure that contributes to easy recovery by solidifying the objective substance with an addition of a specific solvent to the solution after the reaction. Examples thereof include the partial structure disclosed in aforementioned Patent Documents 2 to 6, and in particular, the pseudo solid-phase protective group disclosed in Patent Document 2 described above can be used.

In the method for producing a chimeric molecule of the present invention, since a starting material containing a lipid-soluble anchor as a part of its structure is used, a chimeric molecule containing a lipid-soluble anchor as a part of its structure can be produced. A chimeric molecule not containing a lipid-soluble anchor as a part of its structure may be produced by cleaving off a lipid-soluble anchor from a chimeric molecule containing a lipid-soluble anchor as a part of its structure produced by the method for producing a chimeric molecule of the present invention. Preferably, the lipid-soluble anchor is not cleaved off in the production of the chimeric molecule, but can be cleaved off at any stage. Preferably, the lipid-soluble anchor is not removed under an acidic condition but is removable under a basic condition.

Examples of the lipid-soluble anchor include an organic group having an aromatic hydrocarbon ring having 6 to 14 carbon atoms to which one or more aliphatic hydrocarbon groups having 10 to 40 carbon atoms are bound via a single bond or a linker.

In the aliphatic hydrocarbon group having 10 to 40 carbon atoms, the aliphatic hydrocarbon group may be linear, branched, or cyclic, or may be a mixture thereof. The aliphatic hydrocarbon group may have one or two unsaturated bonds. In the aliphatic hydrocarbon group, one or two methylene carbon atoms may be substituted by ether oxygen atoms.

The aliphatic hydrocarbon group is preferably linear; more preferably linear and free of an unsaturated bond; still more preferably linear and free of an unsaturated bond and an etheric oxygen atom.

The number of carbon atoms of the aliphatic hydrocarbon group having 10 to 40 carbon atoms is preferably from 10 to 30, more preferably from 12 to 28, still more preferably from 14 to 22, and particularly preferably from 16 to 20.

The aliphatic hydrocarbon group having 10 to 40 carbon atoms is preferably a linear alkyl group having 14 to 22 carbon atoms, more preferably a linear alkyl group having 16 to 20 carbon atoms, still more preferably a linear alkyl group having 17 to 19 carbon atoms, and particularly preferably a linear alkyl group having 18 carbon atoms.

The aliphatic hydrocarbon group having 10 to 40 carbon atoms and the aromatic hydrocarbon ring having 6 to 14 carbon atoms are preferably bound to each other via a linker. Examples of the linker include -O-, -C(=O)-, -C(=O)-O-, -O-C(=O)-, -C(=O)-NH-, -NH-C(=O)-, - S-, -S(=O)-, and -S(=O)₂-. Of these, -O-, -C(=O)-, -C(=O)-O-, -O-C(=O)-, -C(=O)-NH-, or -NH-C(=O)- is preferable, and -O- is more preferable.

Examples of the aromatic hydrocarbon ring having 6 to 14 carbon atoms include a benzene ring, a naphthalene ring, an anthracene ring, and a phenanthrene ring. Preferred examples include a benzene ring and a naphthalene ring, and more preferred examples include a benzene ring.

In the aromatic hydrocarbon ring having 6 to 14 carbon atoms, 1 to 5 aliphatic hydrocarbon groups having 10 to 40 carbon atoms may be bound via a single bond or a linker, preferably 1 to 4, more preferably 1 to 3, still more preferably 2 to 3, particularly preferably 3.

Examples of the lipid-soluble anchor include an organic group having a benzene ring to which 1 to 5 aliphatic hydrocarbon groups having 14 to 22 carbon atoms are bound via a -O-linker; preferred examples include an organic group having a benzene ring to which 2 to 3 aliphatic hydrocarbon groups having 16 to 20 carbon atoms are bound via a -O- linker; more preferred examples include an organic group having a benzene ring to which 3 aliphatic hydrocarbon groups having 18 carbon atoms are bound via a -O- linker; and still more preferred examples include an organic group having 3,4,5-tri(n-octadecanyloxy)phenyl.

Examples of the organic group having an aromatic hydrocarbon ring having 6 to 14 carbon atoms to which an aliphatic hydrocarbon group having 10 to 40 carbon atoms is bound via a single bond or a linker include organic groups having the following structures, and preferred examples include an organic group having a structure represented by Formula (I) shown below. 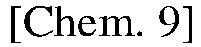 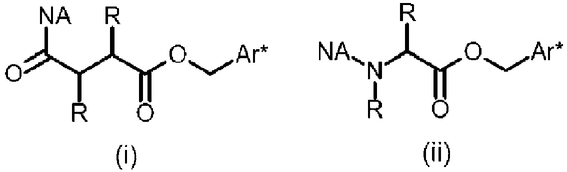
In the formula, Ar* represents an aromatic hydrocarbon ring having 6 to 14 carbon atoms to which an aliphatic hydrocarbon group having 10 to 40 carbon atoms is bound via a single bond or a linker;
R's each independently represent a hydrogen atom or an alkyl group having 1 to 6 carbon atoms; and
NA's each independently represent a position to bond to a structure containing the first nucleic acid or the second nucleic acid.

The alkyl group having 1 to 6 carbon atoms in R may be linear, branched, or cyclic, or may be a mixture thereof. Examples thereof include a methyl group, an ethyl group, an n-propyl group, and an isopropyl group. Preferred examples include a methyl group and an ethyl group, and more preferred examples include a methyl group.

R is preferably a hydrogen atom.

For example, when a carbonyl carbon is positioned at the position to bond to the structure containing the first nucleic acid or the second nucleic acid represented by NA as in Formula (i), it is preferably bound to the oxygen atom at the 3'-position or the 5'-position, preferably the 3'-position, of the deoxyribose or the ribose of a nucleoside.

The hydroxyl group of the first chimeric molecule precursor is preferably a hydroxyl group selected from the group consisting of hydroxyl groups at the 3'-position and the 5'-position of deoxyribose and ribose of a nucleoside, and hydroxyl groups at the 3'-position and the 5'-position of deoxyribose and ribose of DNA and RNA serving as the second nucleic acid; more preferably a hydroxyl group selected from the group consisting of a hydroxyl group at the 5'-position of deoxyribose and ribose of a nucleoside, and a hydroxyl group at the 5'-position of deoxyribose and ribose of DNA and RNA serving as the second nucleic acid.

When the hydroxyl group of the first chimeric molecule precursor is the hydroxyl group at the 5'-position of the deoxyribose or ribose of a nucleoside, the first chimeric molecule precursor may be a first chimeric molecule precursor A in which the oxygen atom at the 3'-position of deoxyribose or ribose of a nucleoside is directly substituted at the position of NA of the lipid-soluble anchor represented by Formula (i).

The first chimeric molecule precursor A has, for example, the following structure. 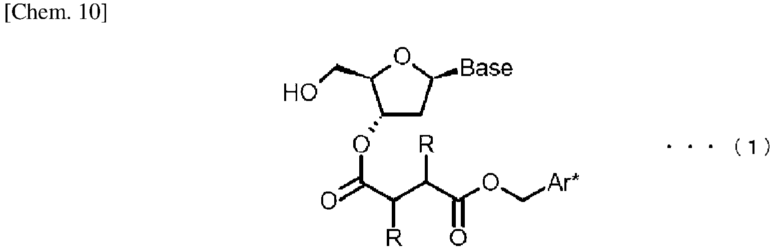
In the formula, Ar* and R have the aforementioned meanings; and
Base represents a base moiety.
The deoxyribose may have an optionally protected hydroxyl group at the 2'-position.

When the hydroxyl group of the first chimeric molecule precursor is a hydroxyl group at the 5'-position of deoxyribose or ribose of DNA or RNA serving as the second nucleic acid, the first chimeric molecule precursor may be a first chimeric molecule precursor B in which the oxygen atom of the hydroxyl group of the first chimeric molecule precursor A may be directly bound to a phosphate site at the 3'-position of deoxyribose or ribose of DNA or RNA serving as the second nucleic acid, or in which the oxygen atom of the hydroxyl group of the first chimeric molecule precursor A is bound to a phosphate site at the 3'-position of deoxyribose or ribose of DNA or RNA serving as the second nucleic acid via one or more first nucleic acids and/or second nucleic acids (which may be a combination of the first nucleic acid and the second nucleic acid).

The first chimeric molecule precursor B has, for example, the following structure. 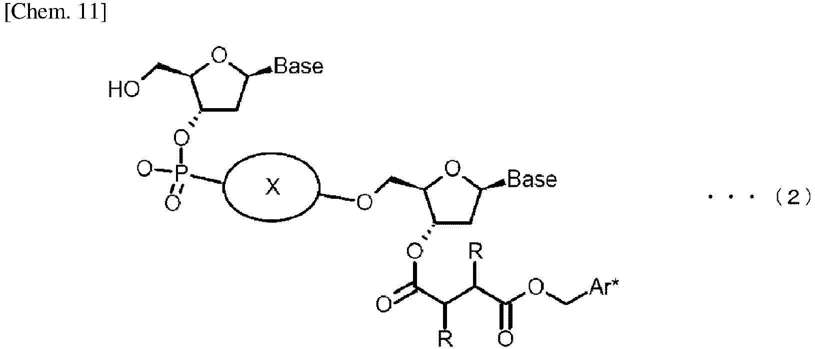
In the formula, Ar*, R, and Base have the aforementioned meanings; and
X represents a single bond, or represents one or more of the first nucleic acid and/or the second nucleic acid through which an oxygen atom bound to X and a phosphorus atom bound to X are held together.
Deoxyriboses may each independently have an optionally protected hydroxyl group at the 2'-position.

When the hydroxyl group of the first chimeric molecule precursor is a hydroxyl group at the 5'-position of deoxyribose or ribose of the DNA or RNA serving as the second nucleic acid, the first chimeric molecule precursor may be a first chimeric molecule precursor C in which the N-terminal of the PNA or PRNA serving as the first nucleic acid having a C-terminal bound to the position of NA of the lipid-soluble anchor represented by Formula (ii) is directly bound to a phosphate site at the 3'-position of deoxyribose or ribose of the DNA or RNA serving as the second nucleic acid, or in which the N-terminal of the PNA or PRNA serving as the first nucleic acid having a C-terminal bound to the position of NA of the lipid-soluble anchor represented by Formula (ii) is bound to a phosphate site at the 3'-position of deoxyribose or ribose of the DNA or RNA serving as the second nucleic acid via one or more first nucleic acids and/or second nucleic acids (which may be a combination of the first nucleic acid and the second nucleic acid).

The first chimeric molecule precursor C has, for example, the following structure. 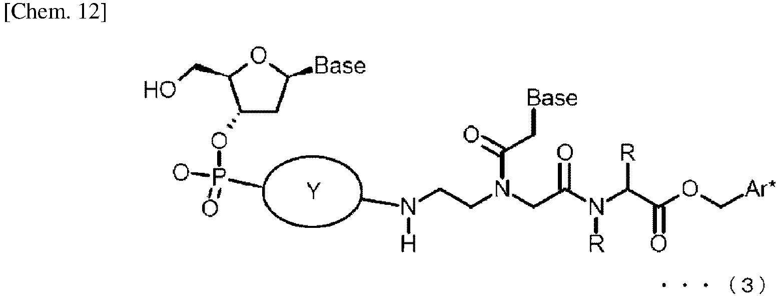
In the formula, Ar*, R, and Base have the aforementioned meanings; and
Y represents a single bond, or represents one or more first nucleic acids and/or second nucleic acids through which a nitrogen atom bound to Y and a phosphorus atom bound to Y are held together.
Deoxyriboses may each independently have an optionally protected hydroxyl group at the 2'-position.

In one or more first nucleic acids and/or second nucleic acids via a bond between an oxygen atom (or nitrogen atom) bound to X (or Y) and a phosphorus atom in X of the first chimeric molecule precursor B and Y of the first chimeric molecule precursor C, the total number of nucleic acids may be from 1 to 70, preferably from 1 to 31, more preferably from 1 to 21, still more preferably from 1 to 10, yet still more preferably from 1 to 5, and particularly preferably from 1 to 3.

The molecular weight of the first chimeric molecule precursor is preferably from 300 to 6000, more preferably from 300 to 5000, and still more preferably from 300 to 4000, in order for the aforementioned lipid-soluble anchor to contribute to easy recovery by solidifying the objective substance with an addition of a specific solvent to the solution after the reaction.

The first chimeric molecule precursor used in this step can be produced, for example, by the methods described in the aforementioned patent documents, in particular, Patent Documents 2 and 7, the methods described in Examples of the present application, or a method similar to these methods.

This step uses an excess amount of the second nucleic acid with respect to the first chimeric molecule precursor and is performed in a solvent inert to the reaction.

As the reaction solvent, a non-polar solvent is preferably used. Examples of the non-polar solvent include halogenated hydrocarbons such as chloroform, dichloromethane, and 1,2-dichloroethane; aromatic hydrocarbons such as benzene, toluene, xylene, and mesitylene; fatty acid esters such as ethyl acetate and isopropyl acetate; aliphatic hydrocarbons such as hexane, pentane, heptane, octane, and cyclohexane; non-polar ethers such as diethyl ether and cyclopentyl methyl ether; and any combinations thereof. Halogenated hydrocarbons are preferable.

A polar solvent may be used in combination as long as the proportion of the non-polar solvent in the entire reaction solvent is 50 vol.% or more. Examples of the polar solvent include nitriles such as acetonitrile and propionitrile; polar ethers such as 1,4-dioxane and tetrahydrofuran; amides such as dimethylformamide, dimethylacetamide, N-methylpyrrolidone, and dimethyl sulfoxide; and any combinations thereof.

For example, a combination of a halogenated hydrocarbon and a nitrile is preferable, and a combination of dichloromethane and acetonitrile is more preferable. In this case, the mixing ratio (vol.%) may be 50:50 to 99:1, preferably 80:20 to 99:1, and more preferably 80:20 to 95:5.

The second nucleic acid used in an excess amount with respect to the first chimeric molecule precursor can be used in an amount of 1.5 to 10 equivalents, preferably 1.6 to 8 equivalents, more preferably 1.8 to 5 equivalents, and still more preferably 2 to 4 equivalents with respect to the number of moles of the first chimeric molecule precursor used.

An activator such as 1H-tetrazole or 4,5-dicyanoimidazole may be used for the purpose of allowing this step to proceed smoothly. When these activators are used, they may be used in an amount of 1 to 25 equivalents, preferably 5 to 20 equivalents, more preferably 8 to 15 equivalents, and still more preferably 8 to 12 equivalents with respect to the number of moles of the first chimeric molecule precursor used.

The reaction temperature and reaction time can be appropriately changed depending on the type and amount of the reagent to be used. For example, stirring may be performed at 0 to 100°C, preferably 15 to 50°C, and more preferably 20 to 30°C for 5 minutes to 24 hours.

The substrate concentration in the reaction can be appropriately changed depending on the type and amount of the reagent to be used. For example, the use amount of solvent can be adjusted such that the concentration of the first chimeric molecule precursor in the reaction solution is from 0.01 to 0.2 mol/L, preferably from 0.02 to 0.1 mol/L, more preferably from 0.025 to 0.08 mol/L, and still more preferably from 0.03 to 0.05 mol/L.

This step may optionally be followed by oxidation or thio-oxidation of the phosphorus atom of the second chimeric molecule precursor.

The oxidation or thio-oxidation of the phosphorus atom can be performed by an ordinary method. For example, reference can be made to the step of converting a phosphite triester bond into a phosphate triester bond or a thiophosphate triester bond in aforementioned Patent Document 7.

For oxidation of the phosphorus atom, a peroxide is preferably used. As the peroxide, tert-butyl hydroperoxide and meta-chloroperbenzoic acid are preferable. As tert-butyl hydroperoxide, a commercially available decane solution or toluene solution can be used as it is or after being diluted 1 to 3 times.

The use amount of the oxidizing agent or the thio-oxidizing agent is, for example, from 1 to 50 moles, preferably from 1 to 10 moles, and more preferably from 2 to 5 moles per mole of the second chimeric molecule precursor or the first chimeric molecule precursor used in the previous step.

The oxidation or thio-oxidation of the phosphorus atom may be performed in so-called one pot following the step of introducing the second nucleic acid without isolation and purification of the second chimeric molecule precursor after the introduction of the second nucleic acid.

After completion of the reaction, the objective substance (second chimeric molecule precursor) may be solidified and recovered by adding a polar solvent to the reaction solution. Since the first chimeric molecule precursor used in the production method of the present invention contains a lipid-soluble anchor, the objective substance (second chimeric molecule precursor) in this step is solidified with an addition of a polar solvent to form a precipitate. Thus, the objective substance of this step can be recovered by filtration.

As the polar solvent, acetonitrile or methanol is preferably used in terms of versatility and cost, and methanol is particularly preferably used.

The amount of the polar solvent to be added to the reaction solution after completion of the reaction in order to recover the objective substance through filtration depends on the substrate concentration in the reaction, and is, for example, from 5 to 50 times, preferably from 5 to 30 times, more preferably from 8 to 20 times, still more preferably from 10 to 15 times the total amount (volume) of the reaction solvent used.

### (A2) Deprotecting of the second chimeric molecule precursor as necessary

The second chimeric molecule precursor formed in the aforementioned step has an optionally protected amino group derived from the introduced second nucleic acid. Because the first nucleic acid is introduced via this amino group in the next step, when an optionally protected amino group is a protected amino group, deprotection of the amino group is necessary.

The conditions for deprotecting the amino group can be appropriately changed depending on the protective group used. Specifically, the method described in aforementioned "Greene's Protective Groups in Organic Synthesis (4th edition, 2006)" can be employed.

For example, when the protective group of the amino group is MMTr, the reaction is performed using an acid in a solvent inert to the reaction.

The acid that can be used is not particularly limited, and examples thereof include halogenoacetic acids such as trichloroacetic acid, trifluoroacetic acid, and dichloroacetic acid; and sulfonic acids such as methanesulfonic acid, trifluoromethanesulfonic acid, and p-toluenesulfonic acid. From the viewpoint of obtaining good results, halogenoacetic acids are preferable, and trichloroacetic acid is particularly preferable.

The use amount of the acid is, for example, from 1 to 100 moles, preferably from 5 to 80 moles, more preferably from 10 to 60 moles, and still more preferably from 20 to 50 moles per number of moles of the second chimeric molecule precursor used.

As in the previous step, after completion of the reaction, the objective substance (deprotected second chimeric molecule precursor) may be solidified and recovered with an addition of a polar solvent to the reaction solution.

The deprotected second chimeric molecule precursor may be directly subjected to the next step, or may be subjected to the next step after eliminating the group capable of leaving from an oxygen atom under a weak base condition, which is represented by LV in the above formula.

For the elimination of the group capable of leaving from the oxygen atom, an ordinary method can be employed. For example, De Napoli et al., Chem. Commun., 2005, 2586 to 2588, and U. Pradere et al., Chem. Rev., 2014, 114, 9154 to 9218 can be referred to for the elimination of the group capable of leaving from the oxygen atom under a weak base condition.

### (A3) Introducing of the first nucleic acid into the second chimeric molecule precursor or the deprotected second chimeric molecule precursor (in this section, also simply referred to as "the second chimeric molecule precursor") (producing of the chimeric molecule)

This step is a step of introducing the first nucleic acid using an amino group of the second chimeric molecule precursor as a starting point. As a result, a chimeric molecule is produced.

In the introduction of the first nucleic acid into the second chimeric molecule precursor in this step, an amide bond is formed by an amidation reaction or a similar reaction between the amino group of the second chimeric molecule precursor and the carboxy group of the first nucleic acid. The first nucleic acid may be one in which one or more first nucleic acids are bound.

As the first nucleic acid, an N-terminal protected PNA monomer represented by an Fmoc-type PNA monomer can be used. The fluorenyl group in Fmoc (fluorenylmethoxycarbonyl) in the Fmoc-type PNA monomer may have one or two substituents at any substitution positions in order to adjust the reactivity upon removal of Fmoc. The substituent group is preferably an electron withdrawing group, and examples thereof include halogen, nitro, cyano, trifluoromethyl, and carbonyl (carboxy, alkyloxycarbonyl, dialkylcarbamoyl, and alkylcarbonyl).

For example, the first nucleic acid used in this step has the following structure. 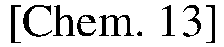 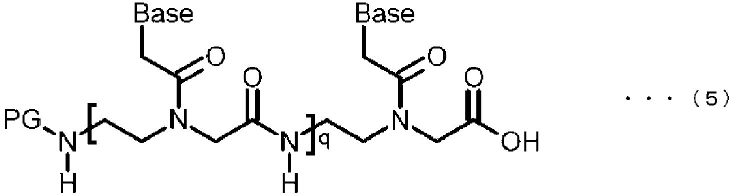
In the formula, q represents an integer of 0 or more;
PG represents a protective group of an adjacent amino group; and
Base represents a base moiety (each of which may be the same or different).

q is preferably from 0 to 40, more preferably from 0 to 30, still more preferably from 0 to 20, yet still more preferably from 0 to 10, and particularly preferably 0.

As the first nucleic acid used in this step, a commercially available compound may be used. In addition, commercially available monomers may be subjected to a normal amidation reaction, whereby a product in which two or more first nucleic acids are bound to each other can be prepared.

As the protective group for the amino group, Fmoc that may have a substituent is preferably used, and Fmoc is more preferably used. It is also possible to use protective groups of the phthalimide type that can be removed using Tr, MMTr, or trityl including DMTr, more preferably MMTr or hydrazine.

This step is performed using an excess amount of the first nucleic acid with respect to the second chimeric molecule precursor in a solvent inert to the reaction, optionally in the presence of a condensing agent.

Examples of the solvent that may be used include, but are not limited to, halogenated hydrocarbons; aromatic hydrocarbons; fatty acid esters; non-polar/polar ethers; nitriles; amides; and any combination thereof. For example, tetrahydrofuran is preferable.

The first nucleic acid used in an excess amount with respect to the second chimeric molecule precursor can be used in an amount of from 1.01 to 5 equivalents, preferably from 1.05 to 3 equivalents, more preferably from 1.1 to 2.5 equivalents, and still more preferably from 1.1 to 2.2 equivalents, with respect to the number of moles of the second chimeric molecule precursor used.

For the purpose of allowing this step to proceed smoothly, a condensing agent may be used.

Examples of the condensing agent include, but are not limited to, dicyclohexylcarbodiimide, 1,1'-carbonyldiimidazole, 1-ethyl-3-(3-dimethylaminopropyl)carbodimide (EDC), 1-[bis(dimethylamino)methylene]-1H-1,2,3-triazolo[4,5-b]pyridinium 3-oxide hexafluorophosphate, (1-cyano-2-ethoxy-2-oxoethylideneaminooxy)dimethylamino-morpholino-carbenium hexafluorophosphate (COMU), diphenylphosphoryl azide, and phosphorus oxychloride. For example, COMU is preferable.

When a condensing agent is used, preferably, when COMU is used as the condensing agent, it may be used in an amount of from 1 to 10 equivalents, preferably from 1.5 to 8 equivalents, more preferably from 2 to 6 equivalents, still more preferably from 2.5 to 5 equivalents, and particularly preferably from 3 to 4.5 equivalents with respect to the number of moles of the second chimeric molecule precursor used.

The carboxy group in the first nucleic acid may be converted to a reactive derivative prior to reaction with the second chimeric molecule precursor. Examples of the reactive derivative of the carboxy group include acid halides obtained by reaction with a halogenating agent such as phosphorus oxychloride or thionyl chloride; mixed acid anhydrides obtained by reaction with isobutyl chloroformate or the like; and active esters obtained by condensation with 1-hydroxybenzotriazole or the like. The reaction of these reactive derivatives with the second chimeric molecule precursor is performed in a solvent inert to the reaction such as halogenated hydrocarbons, aromatic hydrocarbons, and ethers. In some cases, it is advantageous to use an organic base such as triethylamine, N,N-diisopropylethylamine, or N-methylmorpholine in order to allow the reaction to proceed smoothly.

In this step, it may be preferable to use an additive (e.g., 1-hydroxybenzotriazole) for the reaction.

In some cases, it is advantageous to perform the reaction in the presence of an organic base; or an inorganic base such as potassium carbonate, sodium carbonate, or potassium hydroxide in order to allow the reaction to proceed smoothly. For example, N,N-diisopropylethylamine is preferable.

When an organic base is used, preferably, when N,N-diisopropylethylamine is used as the organic base, it can be used in an amount of from 1 to 10 equivalents, preferably from 1.5 to 8 equivalents, more preferably from 1.5 to 5 equivalents, still more preferably from 2 to 4 equivalents, and particularly preferably from 2 to 3 equivalents with respect to the number of moles of the second chimeric molecule precursor used. In addition, it can be used in a molar amount of from 1 to 10 times, preferably from 1.5 to 5 times, more preferably from 2 to 4 times, and still more preferably from 2.5 to 3 times the number of phosphate backbones of the second chimeric molecule precursor.

The reaction temperature and reaction time can be appropriately changed depending on the type and amount of the reagent to be used. For example, stirring may be performed at 0 to 100°C, preferably 15 to 50°C, and more preferably 20 to 30°C for 5 minutes to 24 hours.

The substrate concentration in the reaction can be appropriately changed depending on the type and amount of the reagent to be used. For example, the use amount of solvent can be adjusted such that the concentration of the first chimeric molecule precursor in the reaction solution is from 0.01 to 0.2 mol/L, preferably from 0.02 to 0.1 mol/L, more preferably from 0.025 to 0.08 mol/L, and still more preferably from 0.03 to 0.05 mol/L.

As in the previous step, after completion of the reaction, a polar solvent may be added to the reaction solution to solidify and recover the objective substance (chimeric molecule).

The chimeric molecule obtained in the above step may be further bound to the first nucleic acid or the second nucleic acid via the N-terminal of the introduced first nucleic acid.

In this case, the protective group of the amino group of the introduced first nucleic acid is removed, and the first nucleic acid or the second nucleic acid can be bound using the amino group as a starting point.

The method for removing the protective group for the amino group of the introduced first nucleic acid may be selected depending on the type of the protective group, and the method described in aforementioned "Greene's Protective Groups in Organic Synthesis (4th edition, 2006)" can be employed.

When the protective group for the amino group of the introduced first nucleic acid is Fmoc, for example, a method in which a solution of 2 vol.% of 1,8-diazabicyclo[5.4.0]undec-7-ene (DBU) and 2 vol.% of piperidine, such as a tetrahydrofuran (THF) solution, is allowed to act on the first nucleic acid.

Further introduction of the first nucleic acid into the chimeric molecule can be performed according to aforementioned (3) Introduction of first nucleic acid into second chimeric molecule precursor or deprotected second chimeric molecule precursor (in this section, also simply referred to as "second chimeric molecule precursor") (production of chimeric molecule).

For the introduction of the second nucleic acid, the method described in F. Bergmann, et al., Tetrahedron Lett., 1995, 36(38), 6823 to 6826 or a method similar to this method, or the method described in WO 2017/086397 or a method similar to this method can be employed.

A chimeric molecule not containing a lipid-soluble anchor as a part of its structure can be produced by cleaving off the lipid-soluble anchor from the chimeric molecule thus produced containing the lipid-soluble anchor as a part of its structure.

As described above, it is preferable that the lipid-soluble anchor is not cleaved in the production of the chimeric molecule but can be cleaved at any stage. Preferably, the lipid-soluble anchor is not removed under an acidic condition but is removable under a basic condition.

When the lipid-soluble anchor is a lipid-soluble anchor represented by Formula (I) or (ii), it can be cleaved by the methods described in aforementioned Patent Documents 2 and 7 or a method similar to these methods. For example, the residue of the lipid-soluble anchor is precipitated through the action of an aqueous solution of ammonium hydroxide in a polar solvent, preferably ethanol, and the chimeric molecule that is the objective substance can be isolated from the supernatant. When a group capable of leaving from an oxygen atom under a weak base condition (a group represented by LV) remains at the phosphate site of the introduced second nucleic acid or when a protective group for an amino group of the introduced first nucleic acid (e.g., Fmoc) remains at the phosphate site of the introduced second nucleic acid, a chimeric molecule from which these groups have also left is obtained.

### [Second Aspect of Present Invention]

A method for producing a chimeric molecule according to the second aspect includes the following step (B1):
(B1) Introducing the second nucleic acid via an amino group of a third chimeric molecule precursor having an amino group

Hereinafter, a production method according to the second aspect of the present invention will be described. The production of a DNA-PNA chimeric molecule (a chimeric molecule in which a PNA is fused to the 3'-position side of a DNA) using a PNA as the first nucleic acid and a DNA as the second nucleic acid as necessary will be described as an example.

### (B1) Introducing of the second nucleic acid into a third chimeric molecule precursor having an amino group (hereinafter, also simply referred to as "third chimeric molecule precursor") via the amino group of the third chimeric molecule precursor (producing of chimeric molecule)

This step is a step of introducing the second nucleic acid into the third chimeric molecule precursor using an amino group of the third chimeric molecule precursor as a starting point. As a result, a chimeric molecule is produced.

In addition to having an amino group for introducing the second nucleic acid into the third chimeric molecule precursor, the third chimeric molecule precursor includes a lipid-soluble anchor that contributes to easy recovery by solidifying the objective substance with an addition of a specific solvent to the solution after the reaction. The third chimeric molecule precursor has a partial structure derived from the first nucleic acid, and the amino group of the third chimeric molecule precursor is derived from the first nucleic acid.

The third chimeric molecule precursor having a partial structure derived from the first nucleic acid means that the first nucleic acid, preferably a PNA or a PRNA, more preferably a PNA, is bound to another portion of the third chimeric molecule precursor at the C-terminal, and the N-terminal of the first nucleic acid, preferably a PNA or a PRNA, more preferably a PNA, is exposed. The exposed N-terminal of PNA may be present, for example, as a quaternary ammonium salt. The exposed N-terminal of the first nucleic acid, preferably PNA, is the amino group of the third chimeric molecule precursor and serves as a starting point for introducing the second nucleic acid into the third chimeric molecule precursor.

In the introduction of the second nucleic acid into the third chimeric molecule precursor in this step, the second nucleic acid is subjected to nucleophilic attack from the amino group of the third chimeric molecule precursor to form a bond between the nitrogen atom derived from the amino group of the third chimeric molecule precursor and the phosphorus atom of the DNA serving as the second nucleic acid, preferably the phosphorus atom connected to the 3'- position of the deoxyribose of the DNA. The second nucleic acid may be one in which two or more second nucleic acids are bound.

Preferably, the phosphorus atom of the second nucleic acid subjected to nucleophilic attack is an oxidized pentavalent phosphorus atom. In this case, the pentavalent phosphorus atom is bound to the oxygen atom at the 3'-position of deoxyribose (ribose in the case of RNA), a group capable of finally generating -O⁻, and a hydrogen atom.

As the group capable of finally generating -O⁻, the aspect described in the production method of the first aspect can be employed.

For example, the second nucleic acid used in this step has the following structure. 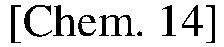 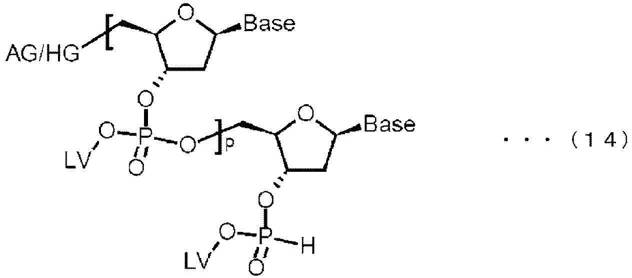
In the formula, p represents an integer of 0 or more;
AG/HG represents an optionally protected amino group or hydroxyl group;
Base represents a base moiety (each of which may be the same or different); and
LV represents a group capable of leaving from an oxygen atom.
Deoxyriboses may each independently have an optionally protected hydroxyl group at the 2'-position.

p is preferably from 0 to 40, more preferably from 0 to 20, still more preferably from 0 to 10, yet still more preferably from 0 to 5, particularly preferably from 0 to 1, and particularly preferably 0.

The optionally protected amino group or hydroxyl group in AG/HG serves as a starting point for further elongating a nucleic acid from the chimeric molecule produced in this step. In order to allow this step to proceed smoothly, a protected amino group or hydroxyl group is preferable. In another aspect, the optionally protected amino group or hydroxyl group in AG/HG is preferably an optionally protected hydroxyl group, more preferably a protected hydroxyl group.

The protective group of the amino group is not particularly limited as long as it is a group that can be removed under an acidic condition, and in the first aspect, a protective group that is acceptable for the optionally protected amino group of the second nucleic acid to be introduced via the hydroxyl group of the first chimeric molecule precursor can be employed.

The protective group for the hydroxyl group is not particularly limited as long as it is a group that can be removed under an acidic condition, and examples thereof include groups described in "Greene's Protective Groups in Organic Synthesis (4th edition, 2006)" written by P. G. M. Wuts and T. W. Greene, and an optionally substituted trityl group is preferable, and an optionally substituted fluorenylmethoxycarbonyl group (Fmoc) is preferable as another aspect.

Examples of the optionally substituted trityl group include a trityl group (Tr), a p-methoxyphenyldiphenylmethyl group (MMTr), and a di (p-methoxyphenyl)phenylmethyl group (DMTr), and more preferred examples include DMTr.

Examples of the optionally substituted fluorenylmethoxycarbonyl group include a fluorenylmethoxycarbonyl group in which the fluorenyl group is substituted with halogen, nitro, cyano, trifluoromethyl, and/or carbonyl (carboxy, alkyloxycarbonyl, dialkylcarbamoyl, alkylcarbonyl).

The second nucleic acid used in this step can be produced, for example, from the compound of aforementioned Formula (4) (the second nucleic acids used in the step A1 of the first aspect) by a known method, the method described in Examples of the present application, or a method similar to these methods.

As the lipid-soluble anchor of the third chimeric molecule precursor, the aspect described in the production method of the first aspect can be employed.

Examples of the organic group having an aromatic hydrocarbon ring having 6 to 14 carbon atoms to which an aliphatic hydrocarbon group having 10 to 40 carbon atoms is bound via a single bond or a linker include organic groups having the following structures, and an organic group having a structure represented by the following Formula (ii) is preferable.

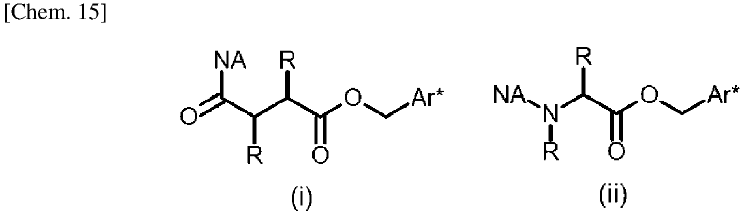

The symbols in the formula are as described above.

For example, when the position to be bound to the structure containing the first nucleic acid or the second nucleic acid represented by NA is a nitrogen atom as represented by Formula (ii), it is preferably bound to the carbonyl carbon of PNA as the first nucleic acid or PRNA to form an amide bond.

The third chimeric molecule precursor may be a third chimeric molecule precursor A in which the partial structure derived from the first nucleic acid of the third chimeric molecule precursor is directly substituted at the position of NA of the lipid-soluble anchor represented by Formula (ii).

The third chimeric molecule precursor A has, for example, the following structure. 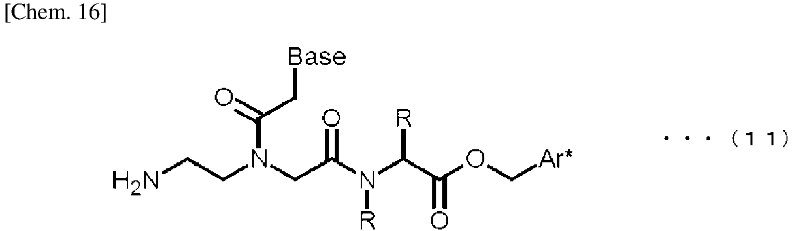
In the formula, Ar* represents an aromatic hydrocarbon ring having 6 to 14 carbon atoms to which an aliphatic hydrocarbon group having 10 to 40 carbon atoms is bound via a single bond or a linker;
R's each independently represent a hydrogen atom or an alkyl group having 1 to 6 carbon atoms; and
Base represents a base moiety.

The third chimeric molecule precursor may be a third chimeric molecule precursor B in which the partial structure derived from the first nucleic acid of the third chimeric molecule precursor and the position of NA of the lipid-soluble anchor represented by Formula (ii) are bound via one or more first nucleic acids and/or second nucleic acids (which may be a combination of the first nucleic acid and the second nucleic acid).

The third chimeric molecule precursor B has, for example, the following structure. 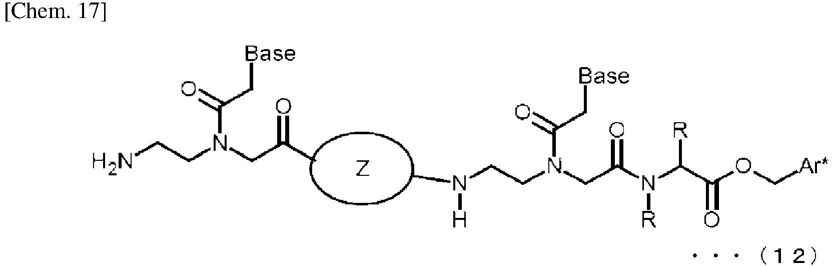
In the formula, Ar*, R, and Base have the aforementioned meanings;
Z represents a single bond, or represents one or more first nucleic acids and/or second nucleic acids through which a nitrogen atom bound to Z and a carbonyl carbon atom bound to Z are held together.
Deoxyriboses may each independently have an optionally protected hydroxyl group at the 2'-position.

The third chimeric molecule precursor may be a third chimeric molecule precursor C in which a hydroxyl group at the 5'-position of deoxyribose or ribose of the nucleoside in which an oxygen atom at the 3'-position is bound at the position of NA of the lipid-soluble anchor represented by Formula (i) and a partial structure derived from the first nucleic acid of the third chimeric molecule precursor are directly bound, or a hydroxyl group at the 5'-position of deoxyribose or ribose of the nucleoside in which an oxygen atom at the 3'-position is bound at the position of NA of the lipid-soluble anchor represented by Formula (i) and a partial structure derived from the first nucleic acid of the third chimeric molecule precursor are bound via one or more first nucleic acids and/or second nucleic acids (which may be a combination of the first nucleic acid and the second nucleic acid).

The third chimeric molecule precursor C has, for example, the following structure. 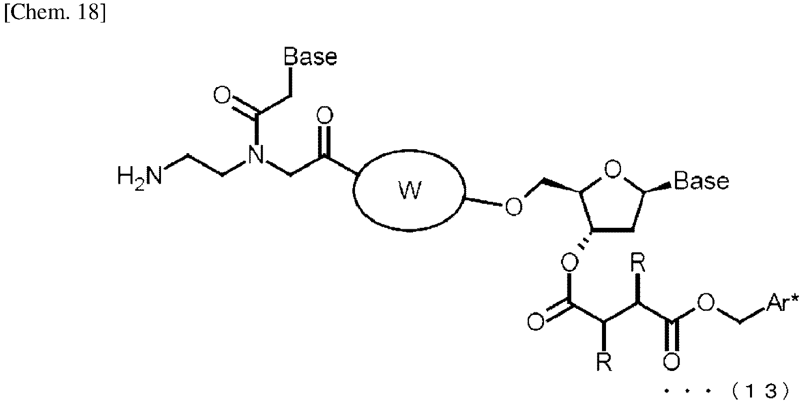
In the formula, Ar*, R, and Base have the aforementioned meanings;
W represents a single bond, or represents one or more first nucleic acids and/or second nucleic acids through which an oxygen atom bound to W and a carbonyl carbon atom bound to W are held together.

Deoxyriboses may each independently have an optionally protected hydroxyl group at the 2'-position.

In one or more first nucleic acids and/or second nucleic acids via a bond between the nitrogen atom (or oxygen atom) bound to Z (or W) and a carbonyl carbon atom in Z of the third chimeric molecule precursor B and W of the third chimeric molecule precursor C, the total number of nucleic acids may be from 1 to 70, preferably from 1 to 31, more preferably from 1 to 21, still more preferably from 1 to 10, yet still more preferably from 1 to 5, and particularly preferably from 1 to 3.

The molecular weight of the third chimeric molecule precursor is preferably from 300 to 6000, more preferably from 300 to 5000, and still more preferably from 300 to 4000, in order for the aforementioned lipid-soluble anchor to contribute to easy recovery by solidifying the objective substance with an addition of a specific solvent to the solution after the reaction.

The third chimeric molecule precursor used in this step can be produced, for example, by the methods described in the aforementioned patent documents, the methods described in Examples of the present application, or methods similar to these methods. The precursor may be produced according to the method described in K. Ogami, et al., Chem. Lett., 2018, 47, 138 to 140.

This step is performed in a solvent inert to the reaction using an excess amount of the second nucleic acid with respect to the third chimeric molecule precursor.

Examples of the solvent that may be used include, but are not limited to, halogenated hydrocarbons; aromatic hydrocarbons; non-polar/polar ethers; nitriles; amides; and any combination thereof.

For example, a combination of a halogenated hydrocarbon and a nitrile is preferable, and a combination of dichloromethane and acetonitrile is more preferable. In this case, the mixing ratio (vol.%) may be 50:50 to 99:1, preferably 80:20 to 99:1, and more preferably 80:20 to 95:5.

The second nucleic acid used in an excess amount with respect to the third chimeric molecule precursor can be used in an amount of from 1.5 to 50 equivalents, preferably from 3 to 30 equivalents, more preferably from 5 to 20 equivalents, and still more preferably from 8 to 15 equivalents with respect to the number of moles of the third chimeric molecule precursor used.

This reaction is a reaction in which the phosphorus atom of the second nucleic acid used in this step is activated by oxidative halogenation, preferably chlorination, to form a bond with the amino group derived from the first nucleic acid in the third chimeric molecule precursor used in this step.

This reaction can be performed under the conditions of the reaction known as Atherton-Todd reaction. For the reaction conditions, reference may be made to, for example, the conditions described in Atherton, F. R. et al., J. Chem. Soc., 1945, 660.

Specifically, the reaction can be performed by using a reagent for oxidatively halogenating the phosphorus atom of the second nucleic acid used in this step and a base for preventing acidification in the reaction system in a solvent inert to the reaction.

Examples of the agent for oxidatively halogenating the phosphorus atom of the second nucleic acid used in this step include carbontetrachloride, sulfuryl chloride, trichloroisocyanuric acid, iodide, and Iodoform. Preferred examples include carbon tetrachloride from the viewpoint of ease of handling.

As the base for preventing acidification in the reaction system, various organic bases and inorganic bases can be used, and tertiary amines such as triethylamine and diisopropylethylamine are preferably used.

To allow the reaction to proceed smoothly, these reagents may be used in an excess amount with respect to the third chimeric molecule precursor used in this step. For example, a reagent for oxidatively halogenating the phosphorus atom of the second nucleic acid and a base for preventing acidification in the reaction system can each be used in an amount of from 1.5 to 50 equivalents, preferably from 3 to 30 equivalents, more preferably from 5 to 20 equivalents, and still more preferably from 8 to 15 equivalents, with respect to the number of moles of the third chimeric molecule precursor used. The base for preventing acidification in the reaction system may be used in an amount equal to or more than that of the reagent for oxidatively halogenating the phosphorus atom of the second nucleic acid in order to prevent acidification in the reaction system.

The reaction temperature and reaction time can be appropriately changed depending on the type and amount of the reagent to be used. For example, stirring may be performed at 0 to 100°C, preferably 15 to 50°C, and more preferably 20 to 30°C for 5 minutes to 24 hours.

The substrate concentration in the reaction can be appropriately changed depending on the type and amount of the reagent to be used. For example, the use amount of the solvent can be adjusted such that the concentration of the third chimeric molecule precursor in the reaction solution is from 0.01 to 0.2 mol/L, preferably from 0.02 to 0.1 mol/L, more preferably from 0.025 to 0.08 mol/L, and still more preferably from 0.03 to 0.05 mol/L.

After completion of the reaction, the objective substance (chimeric molecule) may be solidified and recovered by adding a polar solvent to the reaction solution. Since the third chimeric molecule precursor used in the production method of the present invention contains a lipid-soluble anchor, the objective substance (chimeric molecule) in this step is solidified with an addition of a polar solvent to form a precipitate. Thus, the objective substance of this step can be recovered by filtration.

As the polar solvent, acetonitrile or methanol is preferably used in terms of versatility and cost, and methanol is particularly preferably used.

The amount of the polar solvent to be added to the reaction solution after completion of the reaction in order to recover the objective substance through filtration depends on the substrate concentration in the reaction, and is, for example, from 5 to 50 times, preferably from 5 to 30 times, more preferably from 8 to 20 times, still more preferably from 10 to 15 times the total amount (volume) of the reaction solvent used.

When the polar solvent is added to the reaction solution for recovering the objective substance through filtration and the recovery amount of the objective substance is small, the amount of the polar solvent to be added may be adjusted, preferably increased, or the reaction solution after addition of the polar solvent may be cooled.

The chimeric molecule obtained in the above step may be further bound to the first nucleic acid or the second nucleic acid via the amino group or the hydroxy group at the 5'-position of the introduced second nucleic acid.

In this case, the protective group of the amino group or the hydroxy group at the 5'-position of the introduced second nucleic acid is removed, and the first nucleic acid or the second nucleic acid can be bound using the amino group or the hydroxy group as a starting point.

The method for removing the protective group for the amino group or the hydroxy group of the introduced second nucleic acid may be selected depending on the type of the protective group, and the method described in aforementioned "Greene's Protective Groups in Organic Synthesis (4th edition, 2006)" can be employed.

When the protective group for the hydroxy group of the introduced second nucleic acid is an optionally substituted trityl group, for example, DMTr, it can be removed using an acid in a solvent inert to the reaction.

The acid that can be used is not particularly limited, and examples thereof include halogenoacetic acids such as trichloroacetic acid, trifluoroacetic acid, and dichloroacetic acid; and sulfonic acids such as methanesulfonic acid, trifluoromethanesulfonic acid, and p-toluenesulfonic acid. From the viewpoint of obtaining good results, halogenoacetic acids are preferable, and trichloroacetic acid is particularly preferable.

The use amount of the acid is, for example, from 1 to 100 moles, preferably from 5 to 80 moles, more preferably from 10 to 60 moles, and still more preferably from 20 to 50 moles per number of moles of the chimeric molecule used.

A chimeric molecule not containing a lipid-soluble anchor as a part of its structure can be produced by cleaving off the lipid-soluble anchor from the chimeric molecule thus produced containing the lipid-soluble anchor as a part of its structure.

As described above, it is preferable that the lipid-soluble anchor is not cleaved in the production of the chimeric molecule but can be cleaved at any stage. Preferably, the lipid-soluble anchor is not removed under an acidic condition but is removable under a basic condition.

When the lipid-soluble anchor is a lipid-soluble anchor represented by Formula (I) or (ii), it can be cleaved by the methods described in aforementioned Patent Documents 2 and 7 or a method similar to these methods. For example, the residue of the lipid-soluble anchor is precipitated through the action of an aqueous solution of ammonium hydroxide in a polar solvent, preferably ethanol, and the chimeric molecule that is the objective substance can be isolated from the supernatant. When a group capable of leaving from an oxygen atom under a weak base condition (a group represented by LV) remains at the phosphate site of the introduced second nucleic acid or when a protective group for an amino group of the introduced first nucleic acid (e.g., Fmoc) remains at the phosphate site of the introduced second nucleic acid, a chimeric molecule from which these groups have also left is obtained.

### Examples

The present invention will be described with reference to the following examples, but the present invention is not limited to the examples.

### Production Example A1 (alkylating)

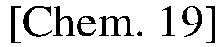

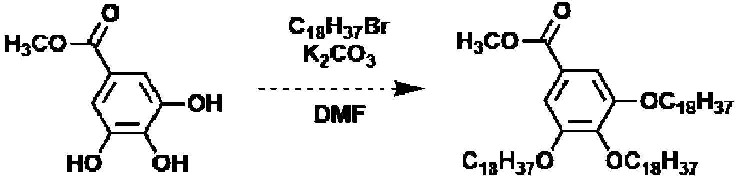

A mixture of methyl gallate (15.0 g, 81.5 mmol), 1-bromooctadecane (89.7 g, 269 mmol), and potassium carbonate (67.2 g, 486 mmol) in DMF (N,N-dimethylformamide) (300 mL) was stirred at 80°C for 1 day. The reaction mixture was cooled to room temperature and diluted with toluene. The obtained material was washed with water. The organic layer was partially concentrated and suspended by addition of methanol. The obtained precipitate was recovered through filtration and the solid was dried under reduced pressure, whereby the target compound (80.1 g, quantitative) was obtained as a white solid.

¹H NMR (600 MHz, CDCl₃) δ 7.24 (s, 2H), 4.00 (td, J = 6.5, 3.4 Hz, 7H), 3.91-3.86 (m, 4H), 1.84-1.69 (m, 7H), 1.45 (td, J = 8.9, 5.1 Hz, 7H), 1.24 (s, 98H), 0.87 (t, J = 7.0 Hz, 11H) ppm.

The NMR data was consistent with those described in an existing document (S. Kim, et al., Eur. J. Chem., 2013, 19, 8615-8620).

### Production Example A2 (LAH reduction)

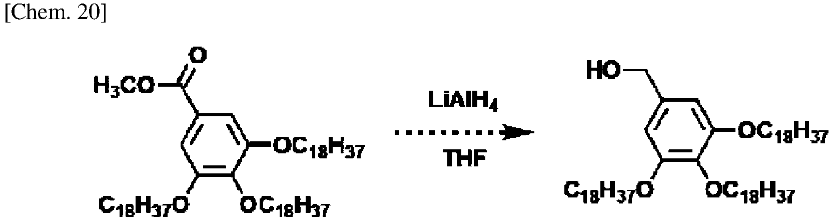

To a solution of LiAlH₄ (1.00 g, 21.1 mmol) in THF (81.0 mL), a solution of methyl ester (5.01 g, 5.32 mmol) in THF (96.0 mL) was added dropwise with cannulation technique at room temperature. After stirring at room temperature for 3 hours, the reaction mixture was cooled in an ice bath and then quenched by addition of water (1.00 mL) followed by 15% aqueous NaOH (3.00 mL). The mixture was warmed to room temperature and stirred for 1 hour. After addition of water (3.00 mL), the mixture was dried over Na₂SO₄ and filtered. The filtrate was concentrated and the crude product was dissolved in dichloromethane (30.0 mL). The product was recrystallized by adding methanol (200 mL) and the precipitate was collected through filtration. The solid was dried under reduced pressure, whereby the target compound (4.55 g, yield 93.6%) was obtained as a white powder.

¹H NMR (400 MHz, CDCl₃) δ 6.54 (s, 2H), 4.58 (s, 2H), 3.94 (dt, J = 15.4, 6.6 Hz, 6H), 1.84-1.66 (m, 6H), 1.46 (d, J = 7.9 Hz, 7H), 1.24 (s, 67H), 0.91-0.82 (m, 9H) ppm.

The NMR data was consistent with those described in an existing document (H. Tamiaki, et al., Bull. Chem. Soc. Jpn., 2001, 74, 733-738).

### Production Example A3 (Introduction of linker)

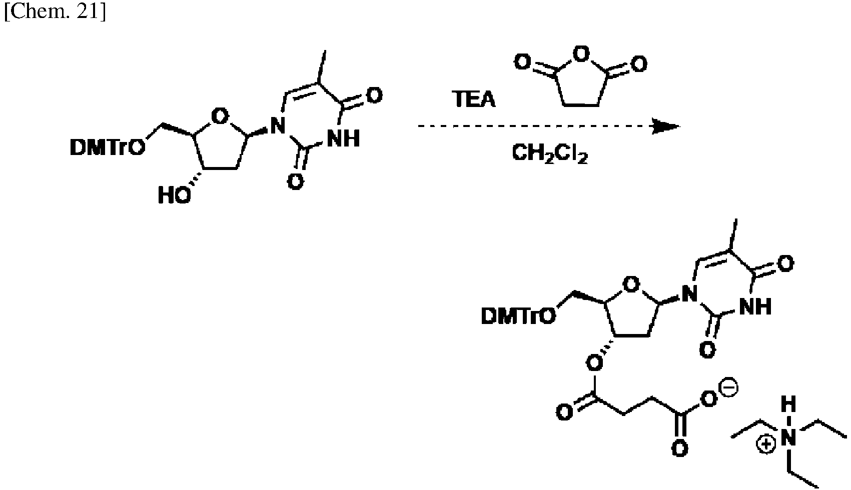

To a solution of 5'-O-DMTr-thymidine (3.00 g, 5.51 mmol) and succinic anhydride (1.10 g, 11.0 mmol) in dichloromethane (8.26 mL), triethylamine (2.79 g, 3.83 mL, 27.6 mmol) was added. After stirring at room temperature for 7.5 hours, the reaction mixture was diluted with dichloromethane and washed with saturated aqueous solution. Washing was performed with NH₄Cl followed by brine. The aqueous phase was extracted twice with dichloromethane. The combined organic layers were dried over Na ₂SO₄ and concentrated, whereby the target compound (4.40 g, quantitative) was obtained as a pale yellow foam.

¹H NMR (600 MHz, CDCl₃) δ 7.61 (s, 1H), 7.38-7.33 (m, 2H), 7.30-7.25 (m, 4H), 7.25-7.18 (m, 6H), 6.84-6.78 (m, 4H), 6.36 (dd, J = 9.3, 5.2 Hz, 1H), 5.41 (d, J = 5.3 Hz, 1H), 5.29 (s, 1H), 4.17 (s, 1H), 3.76 (d, J = 2.6 Hz, 6H), 3.44 (dd, J = 10.2, 2.5 Hz, 1H), 3.39 (dd, J = 10.5, 2.8 Hz, 1H), 3.02 (q, J = 7.3 Hz, 4H), 2.57 (s, 3H), 2.50 (dd, J = 13.6, 5.0 Hz, 1H), 2.40-2.35 (m, 1H), 1.35-1.32 (m, 3H), 1.27 (t, J = 7.3 Hz, 5H) ppm.

The NMR data was consistent with the data described in an existing document (P. Kumar, et al., Nucleosides Nucleotides, 1993, 12, 565-584; C. Johnston, et al., Chemistry-Methods, 2021, 1, 1-8).

### Production Example A4 (Immobilization of nucleoside on anchor)

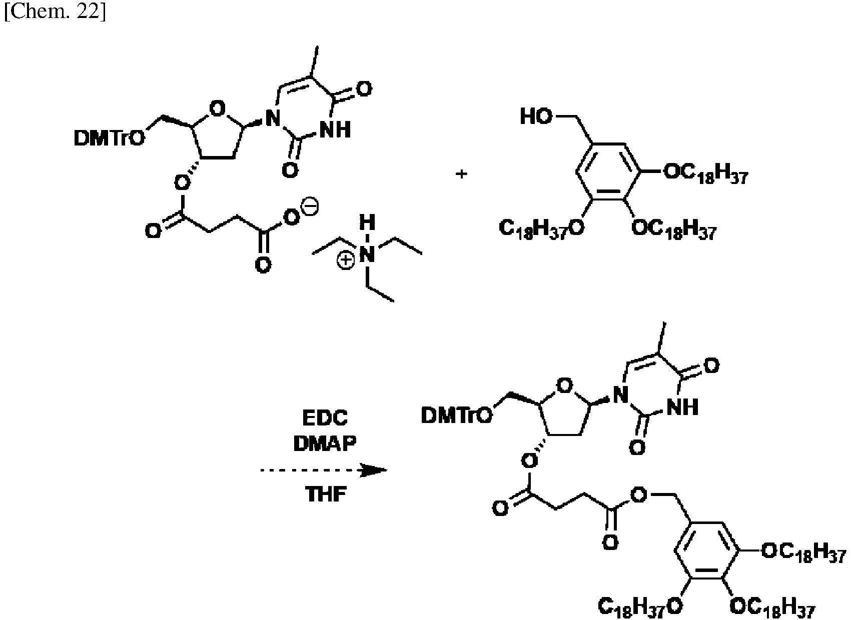

To a solution of a benzyl alcohol derivative (100 mg, 0.109 mmol) in THF (2.00 mL), nucleoside-3'-O-succinate (122 mg, 0.164 mmol), EDC hydrochloride (52.3 mg, 0.273 mmol) and DMAP (4-dimethylaminopyridine) (33.4 mg, 0.273 mmol) was added. After stirring at room temperature for 3 hours, the reaction mixture was suspended by addition of methanol. The obtained precipitate was collected through filtration and rinsed with methanol. The pellet was dried under reduced pressure, whereby the target compound (154 mg, yield 91.7%) was obtained as a white solid.

¹H NMR (600 MHz, CDCl₃) δ 8.19 (s, 1H), 7.60 (d, J = 1.7 Hz, 1H), 7.39-7.34 (m, 2H), 7.32-7.20 (m, 9H), 6.83 (d, J = 8.8 Hz, 4H), 6.52 (s, 2H), 6.42 (dd, J = 8.5, 6.1 Hz, 1H), 5.47 (dd, J = 4.4, 2.7 Hz, 1H), 5.00 (s, 2H), 4.13 (q, J = 2.4 Hz, 1H), 3.94 (d, J = 6.5 Hz, 3H), 3.93-3.88 (m, 3H), 3.78 (s, 6H), 3.50-3.41 (m, 2H), 2.66 (dq, J = 8.5, 5.9 Hz, 4H), 2.48-2.40 (m, 2H), 1.81-1.75 (m, 4H), 1.72 (dt, J = 15.0, 7.6 Hz, 3H), 1.50-1.38 (m, 6H), 1.24 (s, 96H), 0.87 (t, J = 7.1 Hz, 9H) ppm.

¹³ C NMR (151 MHz, CDCl₃) δ 172.10, 171.91, 163.42, 158.87, 153.32, 150.28, 144.25, 138.32, 135.58, 135.31, 135.19, 130.53, 130.18, 128.23, 128.16, 127.35, 113.42, 111.71, 107.11, 87.32, 84.43, 84.05, 75.95, 73.53, 69.22, 67.29, 63.81, 55.35, 37.94, 32.03, 30.43, 29.87, 29.83, 29.77, 29.73, 29.55, 29.51, 29.47, 29.16, 29.03, 26.23, 22.79, 14.23, 11.65 ppm.

HRESIMS calcd. for C ₉₆H₁₅ON₂NaO₁₃, 1562.1030 [M+Na]⁺; found 1562.1060.

### Production Example A5 (Trityl removal; production of first chimeric molecule precursor)

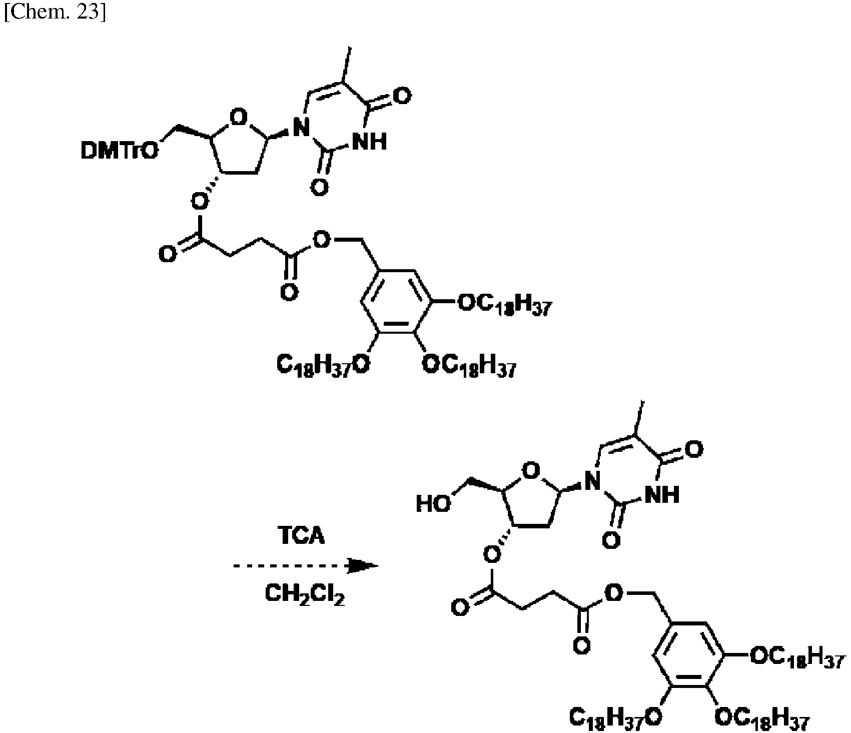

To a cooled solution of the starting material (156 mg, 101 mmol) in chloroform (5.49 mL), 184 mM trichloroacetic acid/dichloromethane (2.74 mL, trichloroacetic acid: 505 mmol) in an ice bath was added. The mixture was stirred at 0°C for 1.5 hours and then warmed to room temperature. After stirring at room temperature for 4.5 hours, the reaction mixture was diluted with methanol. The obtained precipitate was collected through filtration and rinsed with methanol. The solid was dried under reduced pressure, whereby the target compound (117 mg, yield 93.6%) was obtained as a white foam.

¹H NMR (400 MHz, CDCl₃) δ 8.38 (s, 1H), 7.49 (t, J = 1.3 Hz, 1H), 6.52 (s, 2H), 6.18 (dd, J = 8.3, 6.1 Hz, 1H), 5.26 (dt, J = 6.3, 2.4 Hz, 1H), 5.01 (s, 2H), 4.03 (q, J = 2.5 Hz, 1H), 4.01-3.75 (m, 9H), 2.74-2.61 (m, 4H), 2.56 (t, J = 4.9 Hz, 1H), 2.47-2.27 (m, 2H), 1.91 (d, J = 1.2 Hz, 3H), 1.86-1.66 (m, 7H), 1.51-1.38 (m, 7H), 1.24 (s, 97H), 0.91-0.82 (m, 10H) ppm.

¹³ C NMR (101 MHz, CDCl₃) δ 172.11, 172.03, 163.46, 153.29, 150.33, 138.23, 136.48, 130.64, 111.45, 107.16, 86.26, 85.08, 77.31, 75.19, 73.57, 69.24, 67.20, 62.65, 37.16, 32.02, 30.41, 29.86, 29.82, 29.76, 29.72, 29.54, 29.50, 29.47, 29.18, 29.14, 26.22, 22.79, 14.22, 12.68 ppm.

HRESIMS calcd. for C₇₅H₁₃₂N₂NaO₁₁, 1259.9724[M+Na]⁺; found 1259.9734.

### Production Example A6 (Elongation of second nucleic acid)

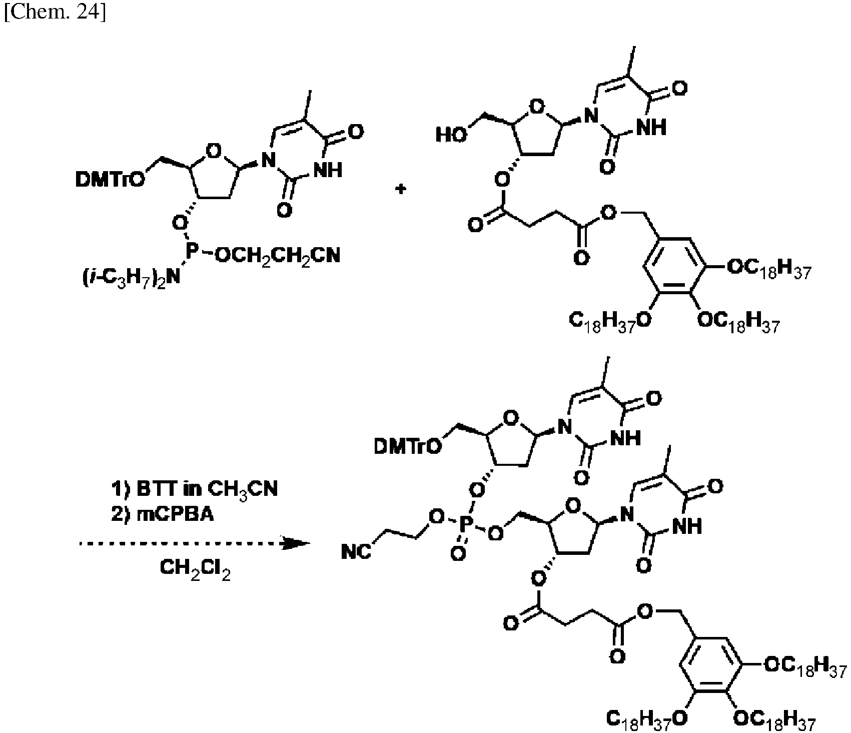

To a dichloromethane solution (1.62 mL) of 3'-O-lipid-soluble anchor modified thymidine (20.0 mg, 16.2 µmol) and 5'-O-DMTr-thymidine phosphoramidite (24.1 mg, 32.4 µmol), an acetonitrile solution (162 µL, 40.5 µmol) of 0.25 M 5-benzylthio-1H-tetrazole (BTT) was added. After the reaction was performed at room temperature for 8 hours, 21.5 mg (81 µmol) of metachloroperbenzoic acid (mCPBA) was added to the reaction solution, and the reaction was performed at room temperature for 1 hour. The reaction solution was suspended by diluting it with methanol. The obtained suspension was filtered to recover the precipitate, and the solid obtained on the filter paper was washed with methanol. Subsequently, the obtained solid was dried overnight under vacuum conditions, whereby dinucleotide as the objective substance was obtained as a white powder (17.9 mg, yield 58.3%).

¹H NMR (600 MHz, CDCl₃) δ 9.26-8.55 (m, 2H), 7.51 (t, J = 12.3 Hz, 1H), 7.43 (d, J = 4.0 Hz, 1H), 7.38-7.27 (m, 9H), 7.27-7.19 (m, 12H), 6.83 (dd, J = 8.5, 3.7 Hz, 6H), 6.52 (d, J = 3.4 Hz, 6H), 6.46-6.33 (m, 1H), 6.33-6.09 (m, 4H), 5.38-5.24 (m, 3H), 5.17 (s, 3H), 5.09-4.90 (m, 6H), 4.40-4.00 (m, 17H), 4.00-3.89 (m, 20H), 3.85 (s, 4H), 3.77 (dd, J = 3.9, 1.7 Hz, 9H), 3.61-3.45 (m, 1H), 3.37 (d, J = 10.3 Hz, 1H), 3.32-3.13 (m, 1H), 2.84-2.73 (m, 4H), 2.73-2.59 (m, 17H), 2.59-2.21 (m, 10H), 1.96-1.85 (m, 14H), 1.85-1.64 (m, 45H), 1.50-1.37 (m, 27H), 1.24 (s, 294H), 0.86 (td, J = 7.0, 1.5 Hz, 31H) ppm.

¹³C NMR (151 MHz, CDCl₃) δ 172.11, 163.85, 163.65, 158.94, 153.33, 150.49, 144.05, 138.34, 136.60, 135.85, 135.73, 135.19, 135.04, 130.51, 130.22, 128.24, 128.17, 127.44, 116.59, 116.44, 116.26, 113.45, 111.88, 111.71, 111.45, 107.15, 87.43, 86.40, 86.27, 85.67, 85.10, 84.39, 82.48, 79.12, 75.17, 74.00, 73.88, 73.58, 73.56, 69.27, 67.69, 67.30, 63.30, 62.61, 62.48, 62.08, 55.38, 39.01, 38.47, 37.16, 36.76, 36.53, 32.02, 30.44, 29.86, 29.82, 29.76, 29.73, 29.55, 29.52, 29.46, 29.18, 29.02, 26.23, 22.78, 19.81, 19.71, 19.67, 14.20, 12.61, 12.53, 11.78 ppm.

³¹P NMR (243 MHz, CDCl₃) δ -1.86, -1.94, -2.09, -2.15 ppm.

HRESIMS calcd. for C₁₀₉H₁₆₆N₅NaO₂₀P, 1920.1790[M+Na]⁺; found 1920.1878.

### Production Example A7 (Trityl removal; production of first chimeric molecule precursor)

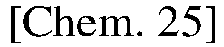

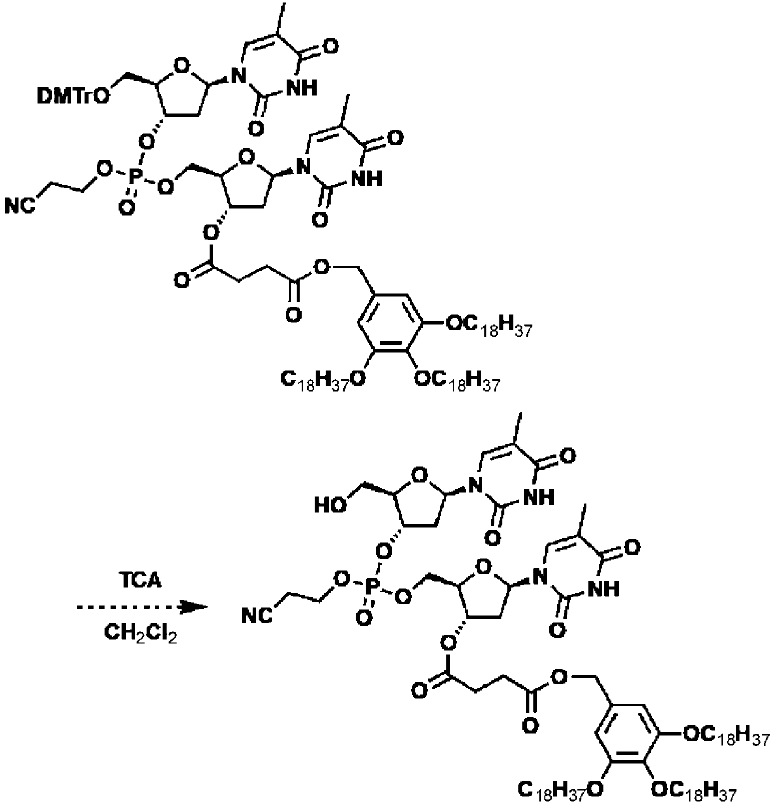

To a solution of the starting material (17.9 mg, 9.43 µmol) in dichloromethane (2.00 mL), a 184 mM trichloroacetic acid/dichloromethane solution (2.00 mL, trichloroacetic acid: 368 µmol) was added on an ice bath. The mixture was stirred at 0°C for 4 minutes, then allowed to return to room temperature and further stirred for 1 hour. The reaction mixture was diluted with methanol. The obtained precipitate was recovered through filter filtration, and the obtained solid was washed with methanol. The solid was dried under reduced pressure, whereby the target compound (16.9 mg, yield > 99.0%) was obtained as a white powder.

¹H NMR (600 MHz, CDCl₃) δ 9.37-8.78 (m, 1H), 7.49 (d, J = 1.3 Hz, 1H), 7.43 (q, J = 2.5 Hz, 1H), 7.37-7.29 (m, 1H), 7.25 (s, 2H), 6.52 (s, 3H), 6.26-6.09 (m, 2H), 5.40-5.24 (m, 1H), 5.17 (td, J = 6.0, 3.0 Hz, 1H), 5.01 (d, J = 3.9 Hz, 3H), 4.42-4.25 (m, 5H), 4.21 (dq, J = 5.4, 2.8 Hz, 1H), 4.15 (t, J = 4.1 Hz, 1H), 4.00-3.89 (m, 9H), 3.85 (s, 3H), 3.28-3.04 (m, 1H), 2.79 (td, J = 6.1, 3.3 Hz, 2H), 2.68 (dh, J = 15.8, 4.2 Hz, 6H), 2.58-2.27 (m, 5H), 1.97-1.84 (m, 8H), 1.81-1.63 (m, 17H), 1.52-1.38 (m, 10H), 1.24 (s, 98H), 0.86 (t, J = 7.0 Hz, 15H) ppm.

¹³C NMR (151 MHz, CDCl₃) δ 172.14, 163.76, 153.33, 150.47, 138.35, 136.67, 135.87, 130.51, 116.59, 111.87, 111.41, 107.12, 86.47, 86.34, 85.70, 82.56, 79.14, 74.02, 73.88, 73.58, 69.27, 67.65, 67.30, 62.61, 62.10, 62.00, 38.46, 37.15, 36.53, 32.02, 30.44, 29.86, 29.82, 29.76, 29.73, 29.55, 29.52, 29.46, 29.04, 26.23, 22.78, 19.86, 19.82, 14.20, 12.66, 12.61, 12.53, 1.10 ppm.

³¹P NMR (243 MHz, CDCl₃) δ -1.86, -1.94 ppm.

HRESIMS calcd. for C₈₈H₁₄₈N₅NaO₁₈P, 1617.0450[M+Na]⁺; found 1617.0467.

### Example A1 (Production of second chimeric molecule precursor)

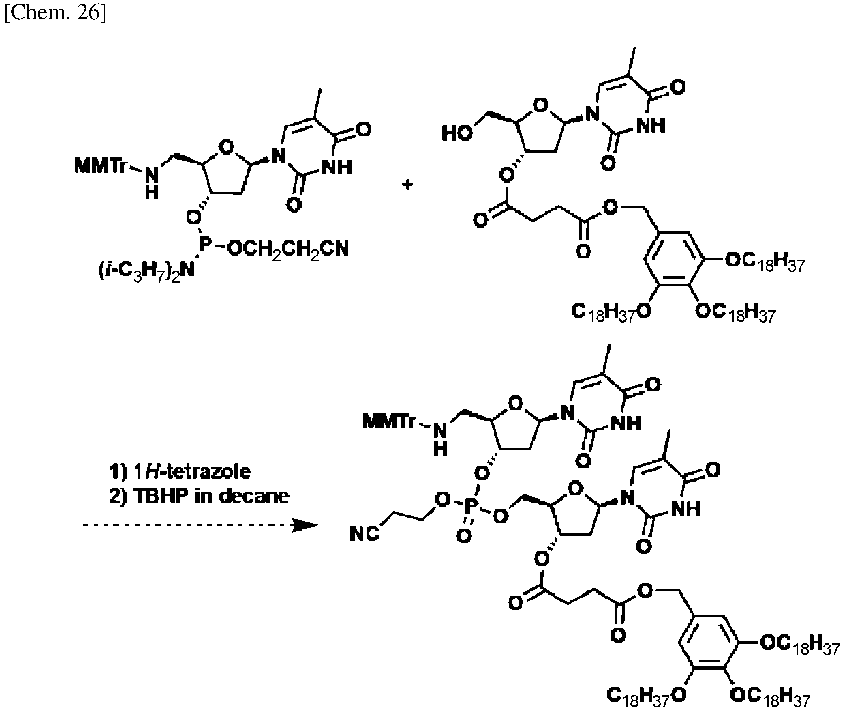

Acetonitrile (43.6 µL) was added to a dichloromethane solution (463 µL) of 3 '-O-lipid-soluble anchor-modified thymidine (20.0 mg, 16.2 µmol). Then, 5'-MMTr-aminated thymidine phosphoramidite (23.1 mg, 32.4 µmol) and 1H-tetrazole (11.3 mg, 162 µmol) were added and caused to react at room temperature for 5 hours. Dichloromethane (200 µL) was added to the reaction solution, and then 5'-MMTr-aminated thymidine phosphoramidite (11.6 mg, 16.2 µmol) and 1H-tetrazole (5.65 mg, 81.0 µmol) were added thereto. After the reaction was performed at room temperature for 6.5 hours, a 5 to 6 M tert-butyl hydroperoxide/decane solution (13.0 µL, 64.8 µmol) was added to the reaction solution, and the reaction was performed at room temperature for 1 hour. The reaction solution was suspended by diluting it with methanol. The obtained suspension was transferred to a centrifuge tube and centrifuged at 3500 rpm for 20 minutes. The obtained precipitate was washed with methanol three times, and the obtained solid was dried overnight under vacuum conditions, whereby dinucleotide as the objective substance was obtained as a white solid (23.3 mg, yield 77.2%).

³¹P NMR (243 MHz, CDCl₃) δ -1.84, -1.95 ppm.

### Example A2 (Production of second chimeric molecule precursor)

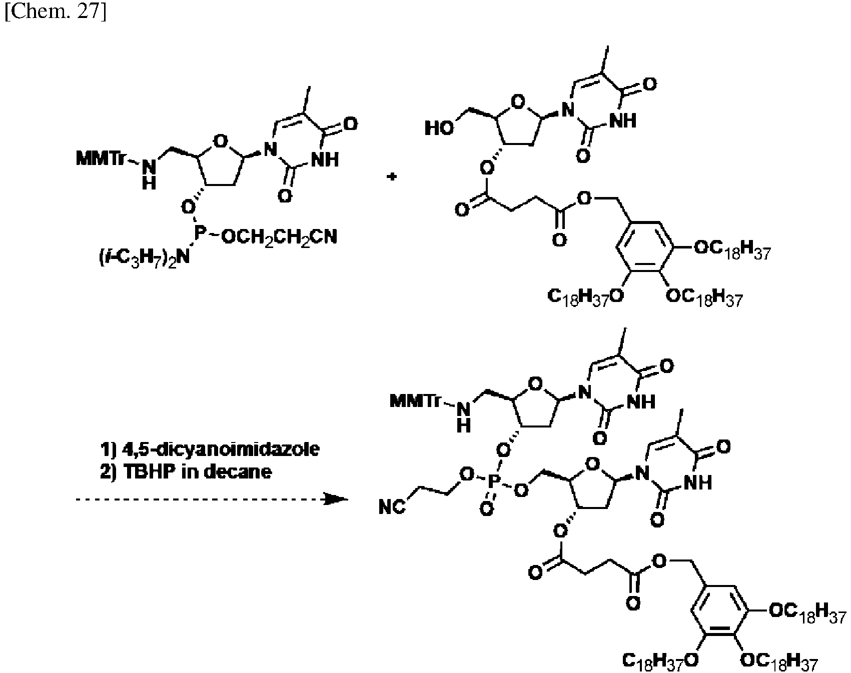

Acetonitrile (40.3 µL) was added to a dichloromethane solution (403 µL) of 3'-O-lipid-soluble anchor-modified thymidine (17.4 mg, 14.1 µmol). Then, 5'-MMTr-aminated thymidine phosphoramidite (20.1 mg, 28.2 µmol) and 4,5-dicyanoimidazole (16.7 mg, 141 µmol) were added, and the mixture was reacted at room temperature for 6 hours. To the reaction solution, 5'-MMTr-aminated thymidine phosphoramidite (20.1 mg, 28.2 µmol) was additionally added, and the mixture was caused to react at room temperature for 3 hours. After completion of the reaction, a 5 to 6 M tert-butyl hydroperoxide/decane solution (11.3 µL, 56.4 µmol) was added to the reaction solution, and the mixture was caused to react at room temperature for 1 hour. The reaction solution was suspended by diluting it with methanol. The obtained precipitate was recovered through filter filtration, and the obtained solid was washed with methanol. The solid was dried under reduced pressure, whereby the target compound (22.9 mg, yield 75.6%) was obtained as a white solid.

¹H NMR (400 MHz, CDCl₃) δ 7.43 (d, J = 8.0 Hz, 3H), 7.33 (d, J = 8.4 Hz, 2H), 7.28 (d, J = 4.4 Hz, 3H), 7.05 (s, 1H), 6.87-6.75 (m, 2H), 6.52 (s, 2H), 6.22 (s, 2H), 5.29 (s, 2H), 5.06-4.93 (m, 3H), 4.41-4.07 (m, 7H), 3.93 (d, J = 9.6 Hz, 7H), 3.76 (d, J = 3.6 Hz, 3H), 2.86 -2.45 (m, 10H), 2.45 -2.20 (m, 4H), 2.16 (s, 4H), 1.90 (d, J = 3.5 Hz, 4H), 1.87-1.67 (m, 11H), 1.45 (s, 10H), 1.24 (s, 92H), 0.87 (t, J = 6.8 Hz, 11H) ppm.

³¹P NMR (162 MHz, CDCl₃) δ -1.95 ppm.

### Example A3 (Deprotection of second chimeric molecule precursor)

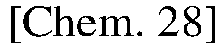

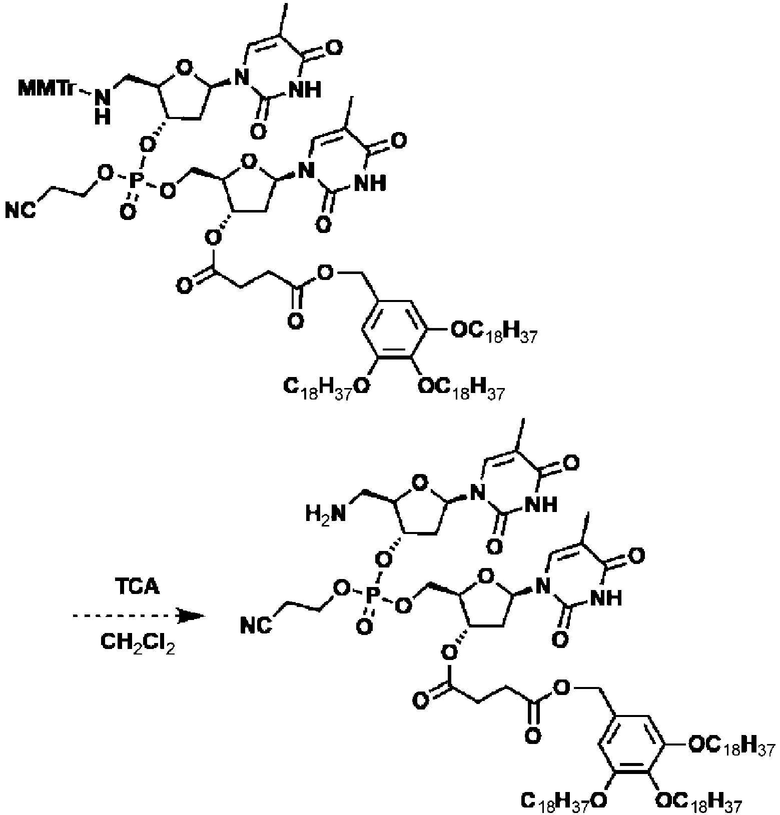

To a solution of the starting material (23.3 mg, 12.5 µmol) in dichloromethane (2.70 mL), a 184 mM trichloroacetic acid/dichloromethane solution (2.70 mL, trichloroacetic acid: 488 µmol) was added on an ice bath. The mixture was stirred at 0°C for 8 minutes, then allowed to return to room temperature and further stirred for 1 hour. The reaction mixture was diluted with methanol. The obtained suspension was transferred to a centrifuge tube and centrifuged at 4000 rpm for 20 minutes. The obtained precipitate was washed with methanol three times, and the obtained solid was dried overnight under vacuum conditions, whereby dinucleotide as the objective substance was obtained as a white solid (8.80 mg, yield 44.2%).

³¹P NMR (162 MHz, CDCl₃) δ -2.02 ppm.

### Example A4 (Introduction of first nucleic acid into second chimeric molecule precursor; production of chimeric molecule)

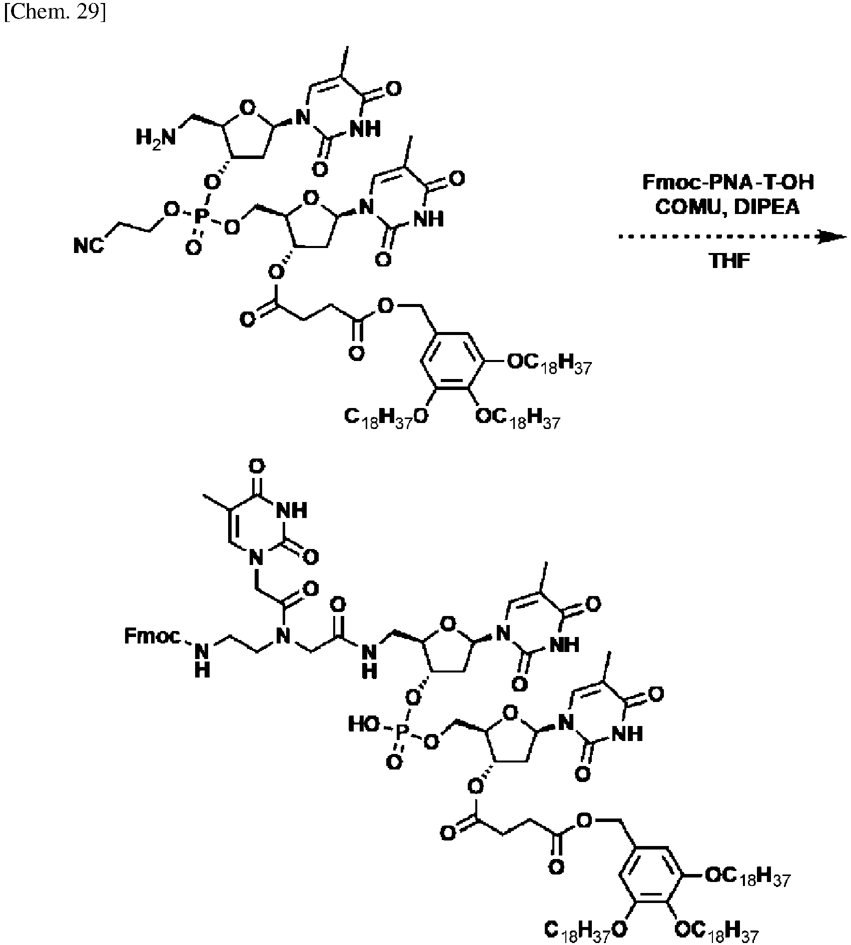

Fmoc-PNA-T monomer (3.35 mg, 6.62 µmol), COMU (7.60 mg, 17.8 µmol), and N,N-diisopropylethylamine (1.78 mg, 2.38 µL, 13.8 µmol) were added in this order to a tetrahydrofuran solution (552 µL) of the starting material (8.80 mg, 5.52 µmol), and the mixture was reacted at room temperature for 20 hours. After completion of the reaction, methanol was added to the reaction solution to suspend the reaction solution. The obtained material was diluted 10 times with acetonitrile, and the obtained suspension was transferred to a centrifuge tube and centrifuged at 4000 rpm for 20 minutes. The obtained precipitate was washed three times with methanol, and the obtained solid was dried overnight under vacuum conditions, whereby a PNA-DNA chimeric molecule was obtained as a white solid (7.90 mg, yield 70.5%).

³¹P NMR (243 MHz, CDCl₃) δ -1.64 ppm.

MALDI-TOF-MS calcd. for C₁₁₁H₁₆₀N₉O₂₃P, 2028.587 [M-H]⁻; found 2028.657.

### Example A5 (Deprotection of chimeric molecule)

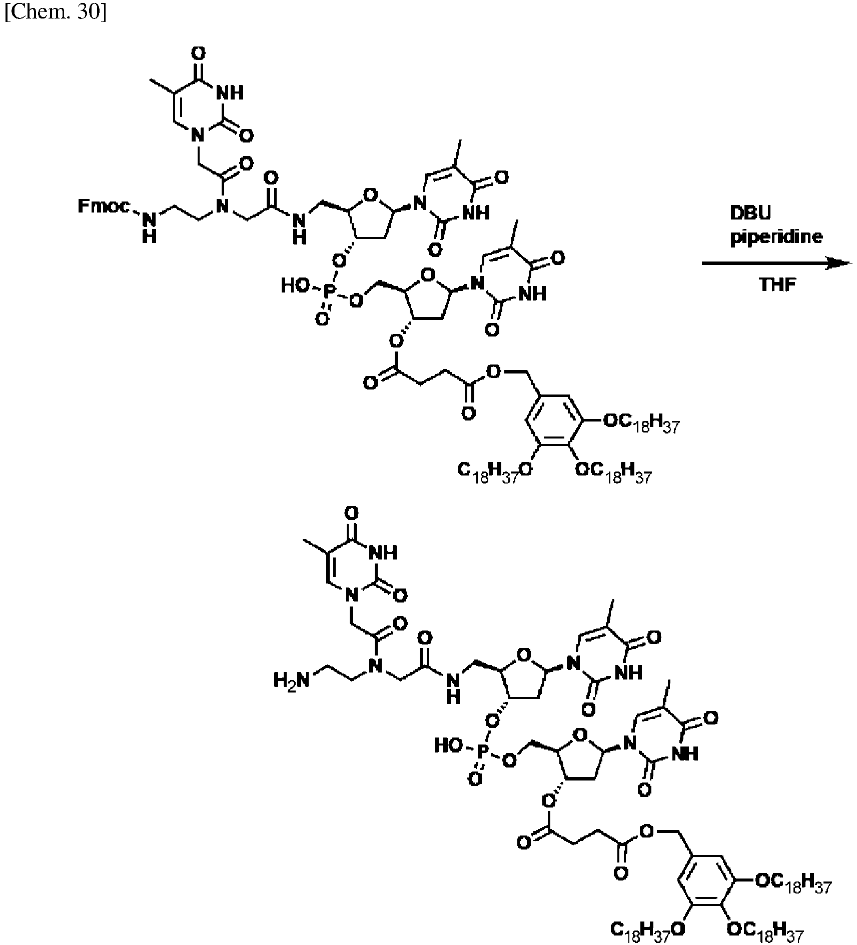

To a tetrahydrofuran solution (500 µL) of the starting material (7.90 mg, 3.89 µmol), a tetrahydrofuran solution (500 µL) of 2% piperidine/2% DBU was added, and the mixture was stirred at room temperature for 1 hour. Until the pH of the reaction solution reached 5, 1 M hydrochloric acid was added dropwise, and then the reaction was terminated. Subsequently, methanol (5.00 mL) was added to the reaction mixture to suspend it, and the obtained suspension was transferred to a centrifuge tube. The suspension was diluted with acetonitrile (34.0 mL) and then centrifuged at 3500 rpm for 20 minutes. The obtained precipitate was washed three times with acetonitrile and the obtained solid was dried overnight under vacuum conditions, whereby a PNA-DNA chimeric molecule was obtained as a white solid (6.80 mg, yield 96.7%).

MALDI-TOF-MS calcd. for C₉₆H₁₅₉N₉O₂₁P, 1806.3443[M-H]⁻; found 1806.446.

### Example A6 (Amino acid introduction into chimeric molecule)

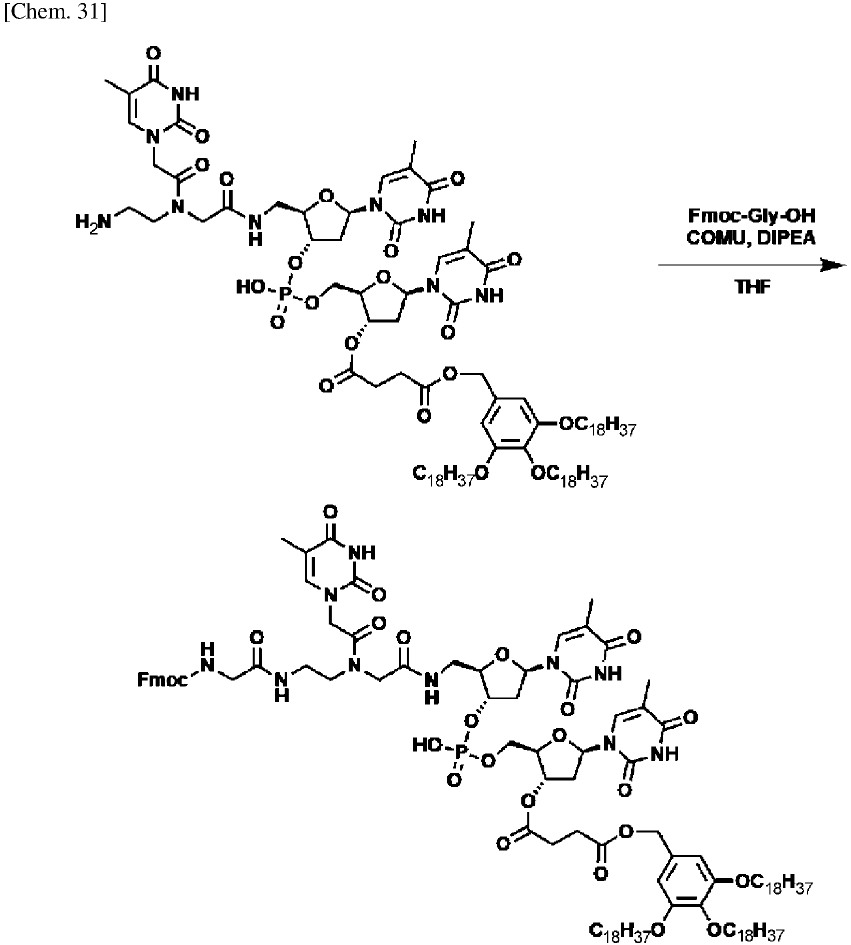

Fmoc-glycine-OH (2.24 mg, 7.52 µmol), COMU (6.42 mg, 15.0 µmol), and N,N-diisopropylethylamine (1.46 mg, 1.95 µL, 11.3 µmol) were added in this order to a tetrahydrofuran solution (376 µL) of the starting material (6.80 mg, 3.76 µmol), and the mixture was caused to react at room temperature for 17.5 hours. After completion of the reaction, methanol was added to the reaction solution to suspend the reaction solution. The obtained material was diluted 10 times with acetonitrile, and the obtained suspension was transferred to a centrifuge tube and centrifuged at 4000 rpm for 20 minutes. The obtained precipitate was washed three times with methanol and the obtained solid was dried overnight under vacuum conditions, whereby a Fmoc-Gly-PNA-DNA chimeric molecule was obtained as a white solid (4.60 mg, yield 58.5%).

MALDI-TOF-MS calcd. for C₁₁₃H₁₇₂N₁₀O₂₄P, 2085.639[M-H]⁻; found 2086.052.

### Example A7 (Cleavage of lipid-soluble anchor from chimeric molecule)

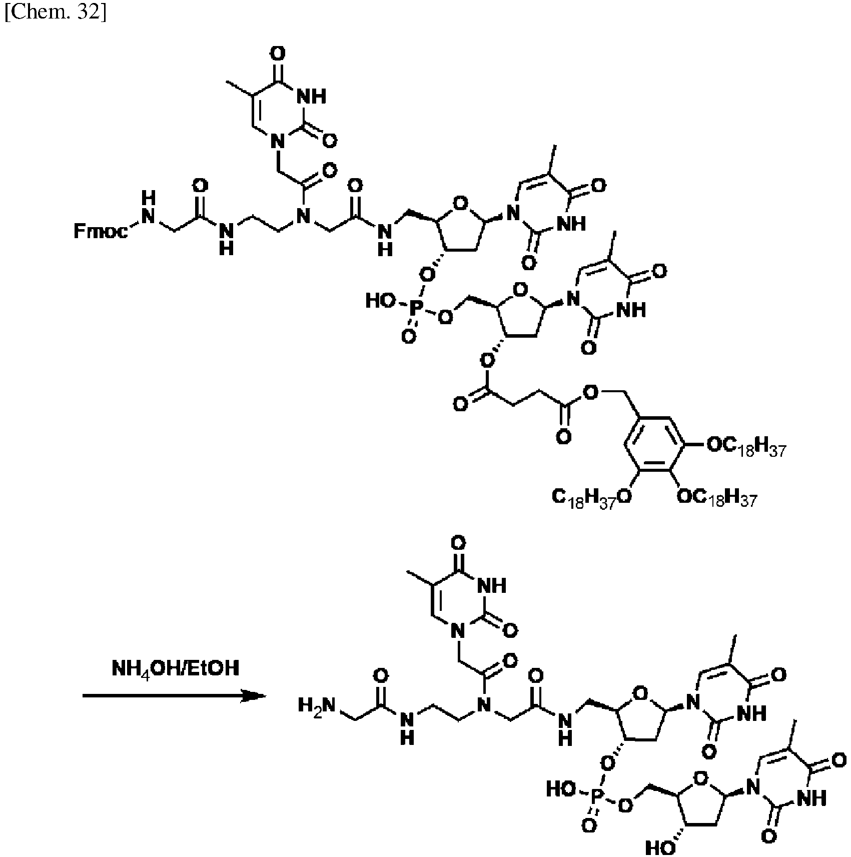

The PNA-DNA chimera (4.60 mg, 2.20 µmol) with a protective group/anchor was suspended in ethanol (400 µL) and 28% aqueous ammonia (1.20 mL) and treated at 80°C for 5 hours. The reaction solution was allowed to return to room temperature, then concentrated and dried. The obtained white solid was suspended in methanol, and the obtained suspension was transferred to a centrifuge tube and centrifuged at 4000 rpm for 15 minutes. The supernatant was recovered, concentrated and dried, then dissolved in super deionized water, and subjected to purity analysis by LC-MS and absorbance measurement by NanoDrop. As a result, the purity of the chimeric molecule N-Gly-T(PNA)TT(DNA)-3' as the objective substance was 63.4%, and the yield was 26.8% (0.59 µmol, ε₂₆₀ = 26,160 M⁻¹·cm⁻¹).

HRESIMS calcd. for C₃₃H₄₄N₁₀O₁₆P, 867.2680[M-H] ; found 867.2758.

### Production Example A8 (Elongation of second nucleic acid, oxidation of phosphorus atom, trityl removal; production of first chimeric molecule precursor)

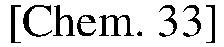

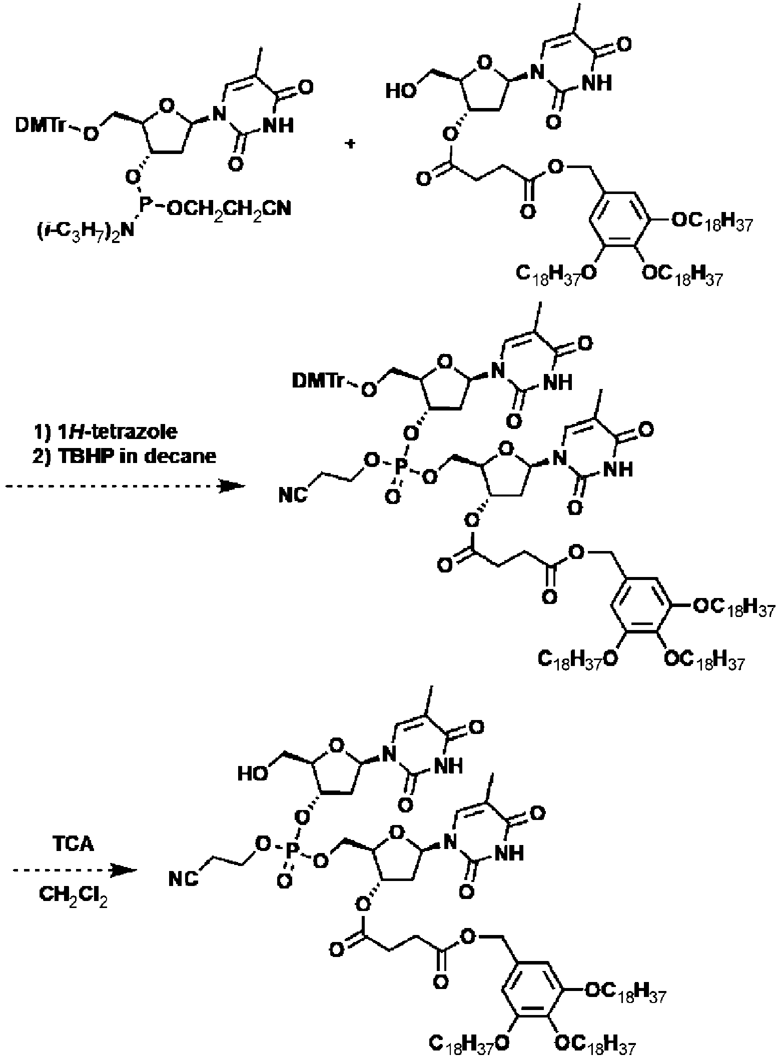

To a mixed solution of 3'-O-lipid-soluble anchor-modified thymidine (108 mg, 87.3 µmol) in dichloromethane (2.49 mL)-acetonitrile (249 µL), 5'-O-DMTr-thymidine phosphoramidite (195 mg, 262 µmol) and 1H-tetrazole (61.2 mg, 874 µmol) were added, and the mixture was caused to react at room temperature for 7.5 hours. A 5 to 6 M tert-butyl hydroperoxide/decane solution (69.8 µL, 349 µmol) was added to the reaction solution, and the mixture was caused to react at room temperature for 1 hour. The reaction solution was suspended by diluting it with methanol. The obtained suspension was transferred to a centrifuge tube and centrifuged at 4000 rpm for 20 minutes. The obtained precipitate was washed three times with methanol and the obtained solid was dissolved in dichloromethane (4.74 mL). After the obtained dichloromethane solution was cooled in an ice bath, a 184 mM trichloroacetic acid/dichloromethane solution (4.74 mL) was added on the ice bath, and the mixture was stirred for 2 minutes. The reaction solution was allowed to return to room temperature, and then further stirred for 1 hour. After completion of the reaction, the reaction solution was suspended by diluting it with methanol. The obtained suspension was transferred to a centrifuge tube and centrifuged at 4000 rpm for 20 minutes. The obtained precipitate was washed three times with methanol, and the obtained solid was dissolved in dichloromethane. Benzene azeotropy was performed three times, and then the obtained solid was dried overnight under vacuum conditions, whereby a dinucleotide form was obtained as a white solid (136 mg, yield 97.8%).

MALDI-TOF-MS calcd. for C₈₈H₁₄₈N₅NaO₁₈P, 1617.045[M+Na]⁺; found 1617.119.

### Production Example A9 (Elongation of second nucleic acid, oxidation of phosphorus atom, trityl removal; production of first chimeric molecule precursor)

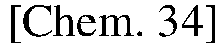

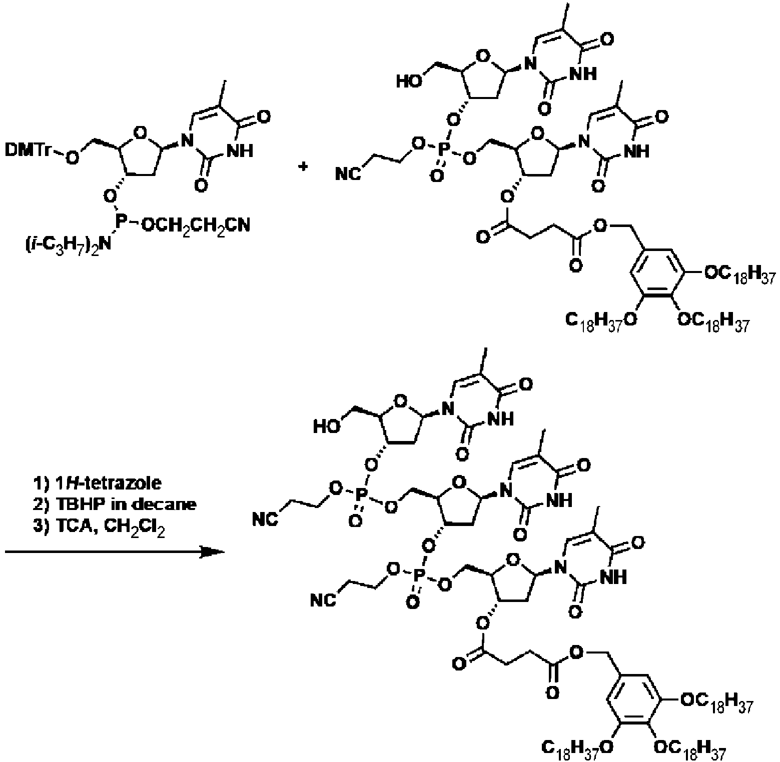

To a mixed solution of 3'-O-lipid-soluble anchor-modified dinucleotide (136 mg, 85.3 µmol) in dichloromethane (2.44 mL)-acetonitrile (244 µL), 5'-O-DMTr-thymidine phosphoramidite (191 mg, 256 µmol) and 1H-tetrazole (59.8 mg, 853 µmol) were added, and the mixture was reacted at room temperature for 5 hours. A 5 to 6 M tert-butyl hydroperoxide/decane solution (68.2 µL, 341 µmol) was added to the reaction solution, and the mixture was reacted at room temperature for 1 hour. The reaction solution was suspended by diluting it with methanol. The obtained suspension was transferred to a centrifuge tube and centrifuged at 4000 rpm for 20 minutes. The obtained precipitate was washed three times with methanol and the obtained solid was dissolved in dichloromethane (4.74 mL). The obtained dichloromethane solution was cooled in an ice bath, then a 184 mM trichloroacetic acid/dichloromethane solution (4.74 mL) was added on the ice bath, and the mixture was stirred for 2 minutes. After the reaction solution was allowed to return to room temperature, 2 to 3 drops of trifluoroacetic acid were added, and the mixture was stirred for 1 hour. After completion of the reaction, the reaction solution was suspended by diluting it with methanol. The obtained suspension was transferred to a centrifuge tube and centrifuged at 4000 rpm for 20 minutes. The obtained precipitate was washed three times with methanol, and the obtained solid was dissolved in dichloromethane. Benzene azeotropy was performed three times, and then the obtained solid was dried overnight under vacuum conditions, whereby a trinucleotide form was obtained as a white solid (82.6 mg, yield 49.5%).

MALDI-TOF-MS calcd. for C₁₀₁H₁₆₄N₈NaO₂₅P₂, 1975.391[M+Na]⁺; found 1975.886.

### Example A8 (Introduction of second nucleic acid into first chimeric molecule precursor: production/deprotection of second chimeric molecule precursor)

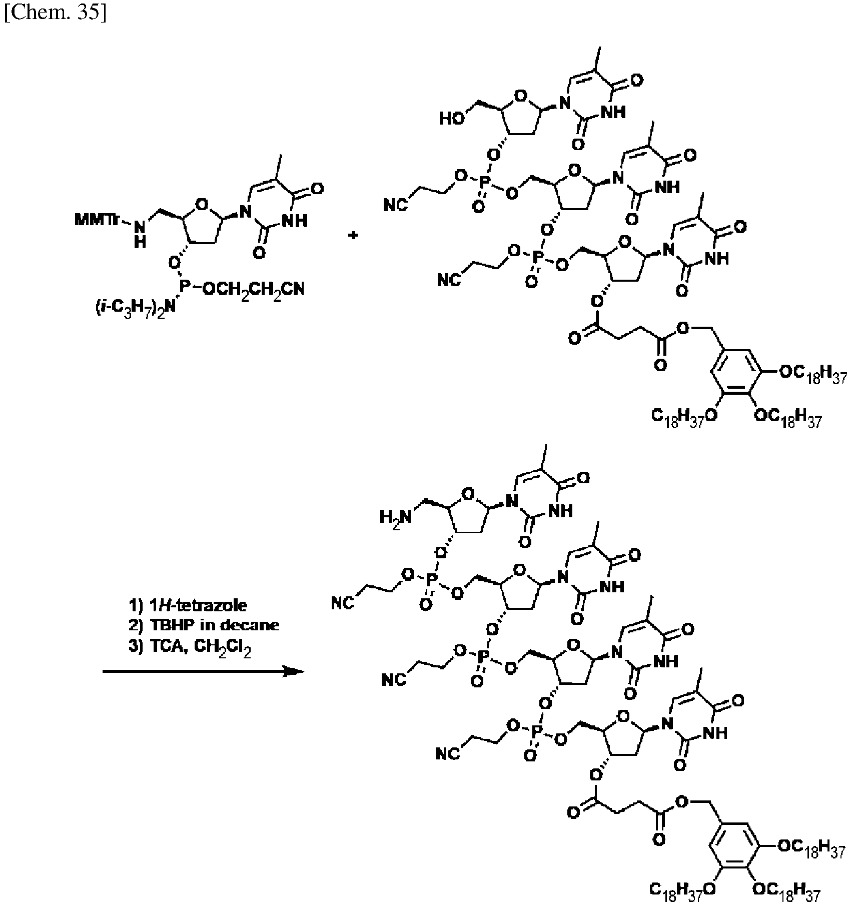

To a mixed solution of 3'-O-lipid-soluble anchor-modified trinucleotide (82.6 mg, 42.3 µmol) in dichloromethane (1.21 mL)-acetonitrile (121 µL), 5'-MMTrNH-thymidine phosphoramidite (90.7 mg, 127 µmol) and 1H-tetrazole (29.6 mg, 423 µmol) were added, and the mixture was caused to react at room temperature for 10 hours. A 5 to 6 M tert-butyl hydroperoxide/decane solution (33.8 µL, 169 µmol) was added to the reaction solution, and the mixture was caused to react at room temperature for 1 hour. The reaction solution was suspended by diluting it with methanol. The obtained suspension was transferred to a centrifuge tube and centrifuged at 4000 rpm for 20 minutes. The obtained precipitate was washed three times with methanol, and the obtained solid was dissolved in dichloromethane (2.00 mL). A 184 mM trichloroacetic acid/dichloromethane solution (10.0 mL) was added to the obtained dichloromethane solution, and the mixture was stirred at room temperature for 45 minutes. After completion of the reaction, the reaction solution was suspended by diluting it with methanol. The obtained suspension was transferred to a centrifuge tube and centrifuged at 4000 rpm for 20 minutes. The obtained precipitate was washed three times with methanol, and the obtained solid was dissolved in dichloromethane. Benzene azeotropy was performed three times, and then the obtained solid was dried overnight under vacuum conditions, whereby a 5'-aminated tetranucleotide form was obtained as a reddish brown solid (75.4 mg, yield 77.2%). It was confirmed through MALDI-TOF-MS analysis that the obtained tetranucleotide was a mixture of one in which all three cyanoethyl groups on the phosphate skeleton were attached, one in which one cyanoethyl group was removed, and two cyanoethyl groups were removed.

MALDI-TOF-MS calcd. for C₁₁₄H₁₈₂N₁₂O₃₁P₃, 2309.684[M+H]⁺; found 2310.708 (all three cyanoethyls attached), C₁₁₁H₁₇₉N₁₁O₃₁P₃, 2256.620[M+H]⁺; found 2257.655 (one cyanoethyl removed), C₁₀₈H₁₇₆N₁₀O₃₁P₃, 2203.566[M+H]⁺; found 2204.544 (two cyanoethyl groups removed).

### Example A9 (Removal of cyanoethyl group)

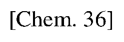

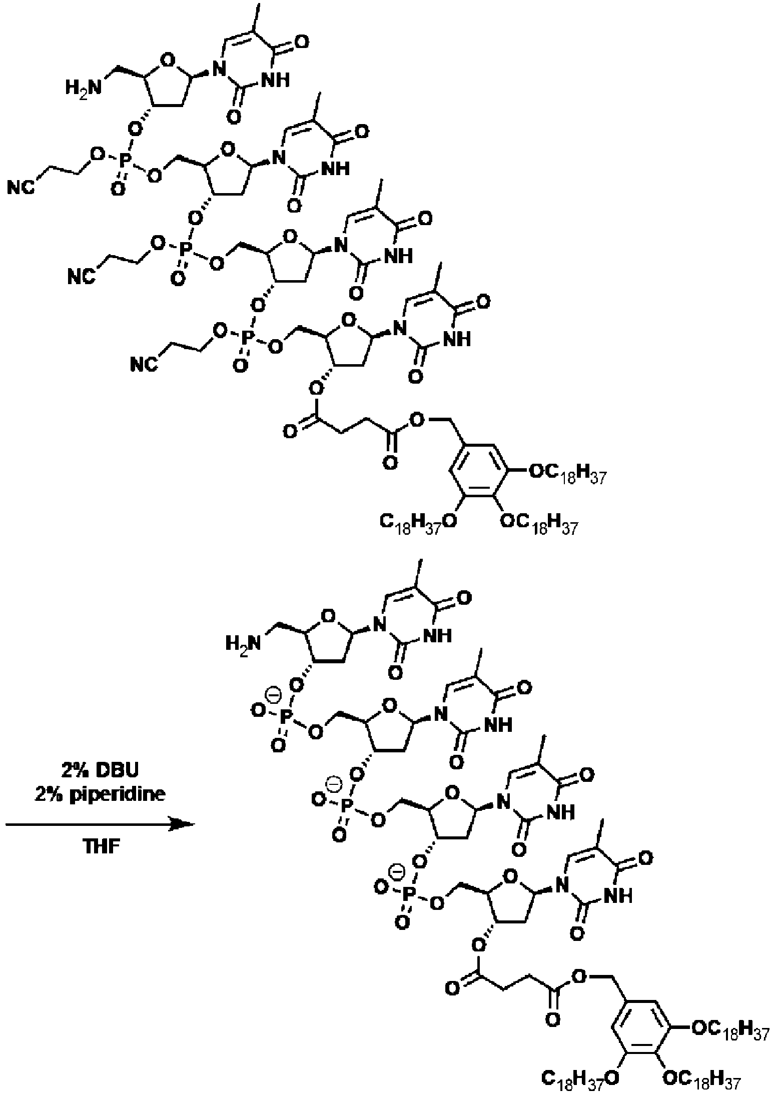

A tetrahydrofuran solution (1.00 mL) of 2% piperidine/2% DBU was added to a tetrahydrofuran solution (1.00 mL) of 5'-aminated tetranucleotide (75.4 mg, 32.7 µmol), and the mixture was stirred at room temperature for 1 hour. Methanol (45.0 mL) was added to the reaction solution to suspend it, and the obtained suspension was transferred to a centrifuge tube and centrifuged at 4000 rpm for 20 minutes. The obtained precipitate was washed three times with methanol, and the obtained solid was dissolved in dichloromethane. Benzene azeotropy was performed three times, and then the obtained solid was dried overnight under vacuum conditions, whereby a decyanoethylated form was obtained as a reddish brown solid (62.7 mg, yield 89.2%).

MALDI-TOF-MS calcd. for C₁₀₅H₁₇₁N₉O₃₁P₃, 2148.477[M-H]⁻; found 2148.571.

### Production Example B1 (esterification)

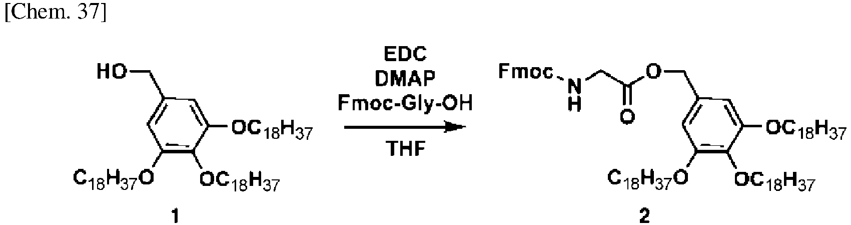

The benzyl alcohol derivative (1) (1.00 g, 1.09 mmol) produced in Production Example A2 was dissolved in tetrahydrofuran (20.0 mL), and then Fmoc-glycine (488 mg, 1.64 mmol), 1-ethyl-3-(3-dimethylaminopropyl)carbodiimide hydrochloride (EDC·HCl) (523 mg, 2.73 mmol) and 4-dimethylaminopyridine (334 mg, 2.73 mmol) were added thereto. The obtained mixture was stirred at room temperature for 20 hours. Methanol (150 mL) was added to the reaction solution, and the obtained precipitate was recovered through suction filtration. The obtained solid was washed with methanol and then dried under reduced pressure, whereby Compound 2 was obtained as a white powder (1.29 g, yield 99.2%).

¹H NMR (600 MHz, CDCl₃) δ 7.77 (d, J = 7.6 Hz, 1.5H), 7.60 (d, J = 7.4 Hz, 1.5H), 7.40 (t, J = 7.4 Hz, 2H), 7.34-7.28 (m, 2H), 6.54 (s, 2H), 5.32-5.27 (m, 0.5H), 5.12-5.04 (m, 2H), 4.41 (d, J = 7.1 Hz, 1.5H), 4.24 (t, J = 7.1 Hz, 0.5H), 4.05 (d, J = 5.6 Hz, 1.5H), 3.94 (ddq, J = 9.4, 6.6, 3.6 Hz, 6H), 1.82-1.69 (m, 6H), 1.49-1.42 (m, 6H), 1.26 (s, 87H), 0.88 (t, J = 7.1 Hz, 9H) ppm.

¹³C NMR (151 MHz, CDCl₃) δ 170.07, 156.41, 153.42, 153.39, 143.92, 141.44, 138.59, 130.07, 127.88, 127.22, 125.22, 120.14, 107.28, 73.58, 69.32, 67.85, 67.40, 47.24, 43.02, 32.08, 30.48, 29.91, 29.87, 29.81, 29.77, 29.58, 29.55, 29.52, 26.27, 26.25, 22.84, 14.26 ppm.

ESI-TOF-MS C₇₈H₁₂₉NNa₂O₇(M+2Na)²⁺calcd.m/z 618.9777, found m/z 619.4417.

### Production Example B2 (Fmoc removal)

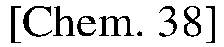

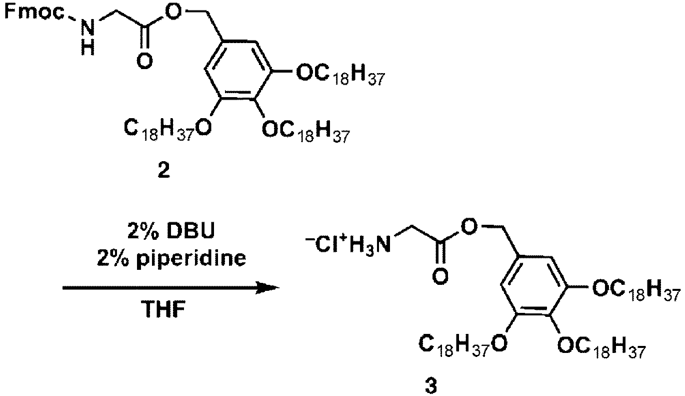

Compound 2 (500 mg, 419 µmol) was dissolved in tetrahydrofuran (21.0 mL), then a 2% piperidine/2% diazabicycloundecene (DBU)/tetrahydrofuran solution (21.0 mL) was added, and the mixture was stirred at room temperature for 3 hours. The reaction solution was adjusted to pH 5 by dropwise addition of 1 M hydrochloric acid. Subsequently, acetonitrile (200 mL) was added to precipitate the reaction product. The obtained precipitate was recovered through suction filtration. The obtained solid was washed with acetonitrile (200 mL) and then dried under reduced pressure, whereby Compound 3 was obtained as a white solid (429 mg, yield 99.2%).

¹H NMR (600 MHz, CDCl₃) δ 8.74 (s, 2H), 6.50 (s, 2H), 5.06 (s, 2H), 4.00 (s, 1H), 3.90 (q, J = 6.6 Hz, 6H), 1.81-1.68 (m, 9H), 1.44 (dp, J = 12.2, 7.0 Hz, 7H), 1.25 (d, J = 4.1 Hz, 80H), 0.90-0.85 (m, 10H) ppm.

ESI-TOF-MS C₆₃H₁₂₀NO₅(M+H)⁺calcd. m/z 970.9161, found m/z 970.9318.

### Production Example B3 (Immobilization of first nucleic acid on anchor)

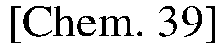

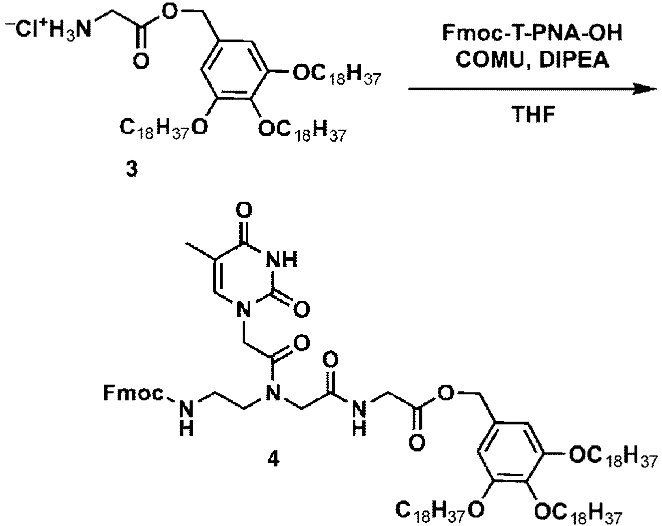

Compound 3 (200 mg, 199 µmol) was dissolved in tetrahydrofuran (19.9 mL), Fmoc-PNA(Thymine)-OH (202 mg, 398 µmol), ethyl 2-cyano-2-((dimethylimino)(morpholino)methoxyimino)acetate hexafluorophosphate (COMU) (256 mg, 597 µmol) and diisopropylethylamine (77.2 mg, 103 µL, 597 µmol) were added to the solution, and the mixture was stirred at room temperature for 6 hours. Acetonitrile (100 mL) was added to the reaction solution, and the resulting precipitate was recovered through suction filtration. The obtained solid was washed with acetonitrile (200 mL) and then dried under reduced pressure, whereby Compound 4 was obtained as a pale brown solid (281 mg, yield 96.9%).

¹H NMR (600 MHz, CDCl₃) δ 8.37 (s, 0.4H), 8.13 (s, 0.6H), 7.74 (dd, J = 7.9, 4.4 Hz, 2H), 7.60 (dd, J = 16.0, 7.5 Hz, 2H), 7.37 (t, J = 7.5 Hz, 2H), 7.29 (dd, J = 14.9, 7.5 Hz, 2H), 7.05 (s, 0.6H), 6.94 (s, 0.3H), 6.77 (s, 0.5H), 6.60-6.54 (m, 0.5H), 6.49 (s, 2H), 5.07 (s, 1H), 5.01 (s, 0.5H), 4.44 (s, 1H), 4.38 (d, J = 7.4 Hz, 1.5H), 4.22-4.15 (m, 1H), 4.08-4.02 (m, 3H), 3.93-3.88 (m, 6H), 3.63 (s, 1H), 3.43 (d, J = 4.6 Hz, 1H), 2.16 (s, 0.2H), 2.00 (s, 0.8H), 1.82 (s, 2H), 1.78-1.68 (m, 7H), 1.57 (s, 7H), 1.43 (d, J = 7.7 Hz, 7H), 1.24 (s, 80H), 0.87 (t, J = 7.1 Hz, 10H) ppm. ESI-TOF-MS C₈₉H₁₄₄N₅O₁₁(M+H)⁺calcd.m/z 1460.1545, found m/z 1460.2759.

### Production Example B4 (Production of third chimeric molecule precursor)

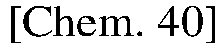

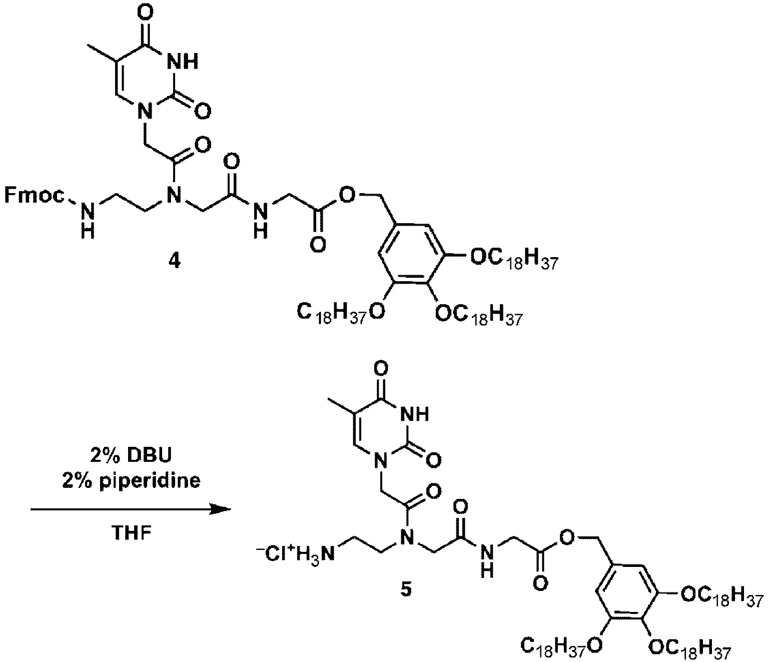

Compound 4 (250 mg, 171 µmol) was dissolved in tetrahydrofuran (17.1 mL), then a 2% piperidine/2% diazabicycloundecene (DBU)/tetrahydrofuran solution (17.1 mL) was added, and the mixture was stirred at room temperature for 3 hours. The reaction solution was adjusted to pH 5 by dropwise addition of 1 M hydrochloric acid. Subsequently, acetonitrile (150 mL) was added to precipitate the reaction product. The obtained precipitate was recovered through suction filtration. The obtained solid was washed with acetonitrile (150 mL) and then dried under reduced pressure, whereby Compound 5 was obtained as a white solid (207 mg, yield 95.0%).

¹H NMR (600 MHz, CDCl₃) δ 10.49 (br, 0.5H), 8.82 (br, 0.5H), 8.53 (m, 1H), 7.91 (m, 1.5H), 7.14 (m, 0.5H), 6.61-6.43 (m, 2H), 4.97 (s, 1.8H), 4.70 (m, 0.6H), 4.58 (s, 0.2H), 4.55-4.32 (m, 1.4H), 4.06 (m, 2H), 3.97-3.86 (m, 6.4H), 3.66 (m, 0.6H), 3.30 (m, 1.8H), 3.13 (m, 0.2H), 2.00 (d, J = 8.4 Hz, 1H), 1.87-1.70 (m, 11H), 1.42 (d, J = 15.3 Hz, 6H), 1.33-1.22 (m, 85H), 0.89-0.83 (m, 9H) ppm.

ESI-TOF-MS C₇₄H₁₃₄N₅O₉(M+H)⁺calcd. m/z 1237.0176, found m/z 1237.0181.

### Production Example B5 (Preparation of second nucleic acid to be introduced)

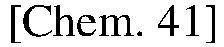

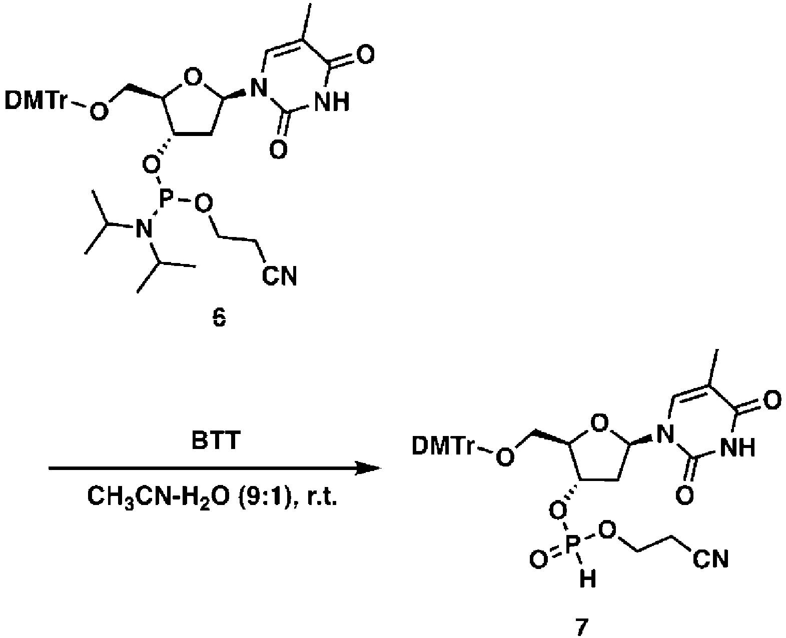

In acetonitrile (22.3 mL), 5'-O-DMTr-thymidine 3'-O-phosphoramidite (1.00 g, 1.34 mmol) was dissolved, and then super deionized water (2.48 mL) was added thereto. Subsequently, a 0.25 M 5-(benzylthio)-1H-tetrazole/acetonitrile solution (10.7 mL, 2.68 mmol) was added, and the mixture was stirred at room temperature for 1 hour. The reaction solution was diluted with ethyl acetate (150 mL) and washed three times with saturated aqueous sodium hydrogen carbonate solution (150 mL) and once with saturated brine (150 mL). The organic layer was dried over anhydrous sodium sulfate and concentrated with a rotary evaporator, whereby Compound 7 was obtained as a white foamy solid (980 mg, > 99%).

¹H NMR (600 MHz, CDCl₃) δ 9.30 -9.14 (m, 1H), 7.45-7.41 (m, 1.5H), 7.29-7.26 (m, 2H), 7.23-7.14 (m, 7H), 6.75 (dq, J = 7.6, 1.0 Hz, 4H), 6.36 (ddd, J = 8.8, 5.5, 1.4 Hz, 1H), 6.22 (d, J = 11.2 Hz, 0.5H), 5.20-5.10 (m, 1H), 4.23-3.99 (m, 3H), 3.69 (d, J = 0.7 Hz, 6H), 3.45 (td, J = 10.8, 3.0 Hz, 1H), 3.31 (ddd, J = 10.7, 3.9, 2.6 Hz, 1H), 2.67 (t, J = 6.1 Hz, 1H), 2.61-2.47 (m, 2H), 2.37 (dtd, J = 14.3, 8.1, 5.9 Hz, 1H), 1.33 (t, J = 1.0 Hz, 3H) ppm.

³¹P NMR (243 MHz, CDCl₃) δ 7.56 ppm.

ESI-TOF-MS C₃₄H₃₆N₃NaO₉P (M+Na)⁺calcd. m/z 684.2081, found m/z 684.2104.

### Example B1 (Introduction of second nucleic acid into third chimeric molecule precursor: production of chimeric molecule)

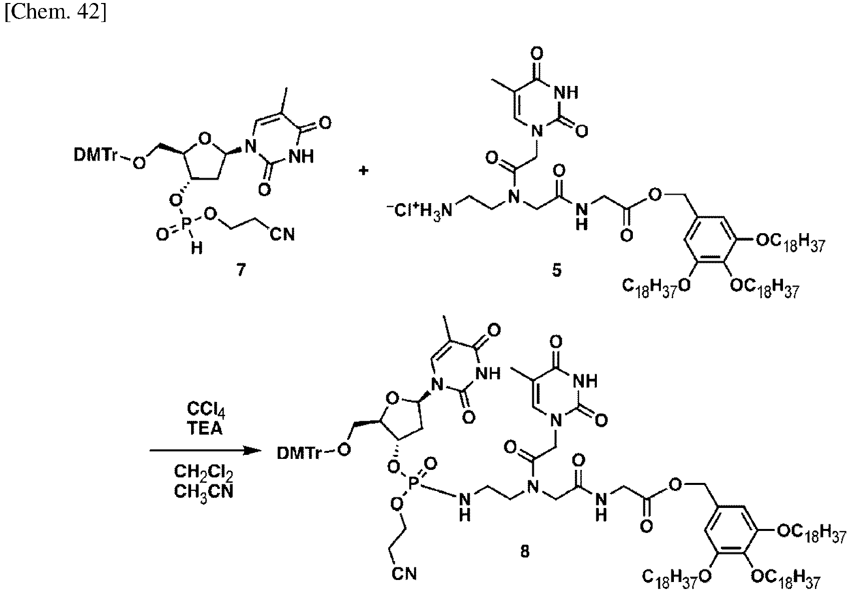

Compound 5 (20.0 mg, 15.7 µmol) was suspended in dichloromethane (449 µL)-acetonitrile (45.0 µL), and then Compound 7 (104 mg, 157 µmol), carbon tetrachloride (24.1 mg, 15.1 µL, 157 µmol), and triethylamine (15.9 mg, 21.8 µL, 157 µmol) were added. The obtained solution was stirred at room temperature for 6 hours, then methanol (13.0 mL) was added to the reaction solution, and the obtained suspension was transferred to a centrifuge tube. The supernatant was removed through centrifugation at 3500 rpm for 7 minutes, and the precipitate was recovered. The precipitate was suspended again in methanol (13.0 mL) and centrifuged, and the supernatant was removed. This operation was repeated three times. The precipitate was dried under reduced pressure, whereby Compound 8 was obtained as a white powder (25.0 mg, yield 83.9%).

¹H NMR (400 MHz, CDCl₃) δ 7.50 (br, 0.6H), 7.35 (s, 0.4H), 7.30-7.25 (m, 3H), 7.18-7.14 (m, 3H), 7.03 (br, 0.5H), 6.84-6.80 (m, 3.5H), 6.55-6.49 (m, 2H), 6.18 (br, 1H), 5.29 (s, 6H), 5.11 (br, 1H), 5.05-5.02 (m, 2H), 4.29-4.19 (m, 4H), 4.09-4.03 (m, 2.5H), 3.95-3.90 (m, 8H), 3.79-3.77 (m, 6H), 2.77 (t, J = 4 Hz, 2H), 2.51 (br, 1H), 1.89-1.86 (m, 4H), 1.79-1.68 (m, 10H), 1.44-1.43 (m, 6.5H), 1.28-1.24 (m, 90H), 0.88-0.85 (m, 10H) ppm.

³¹P NMR (162 MHz, CDCl₃) δ 9.75, 9.55 ppm.

ESI-TOF-MS C₁₀₈H₁₆₇KN₈NaO₁₈P (M+K+Na)²⁺calcd. m/z 979.0847, found m/z 980.9728.

### Example B2 (Deprotection of chimeric molecule)

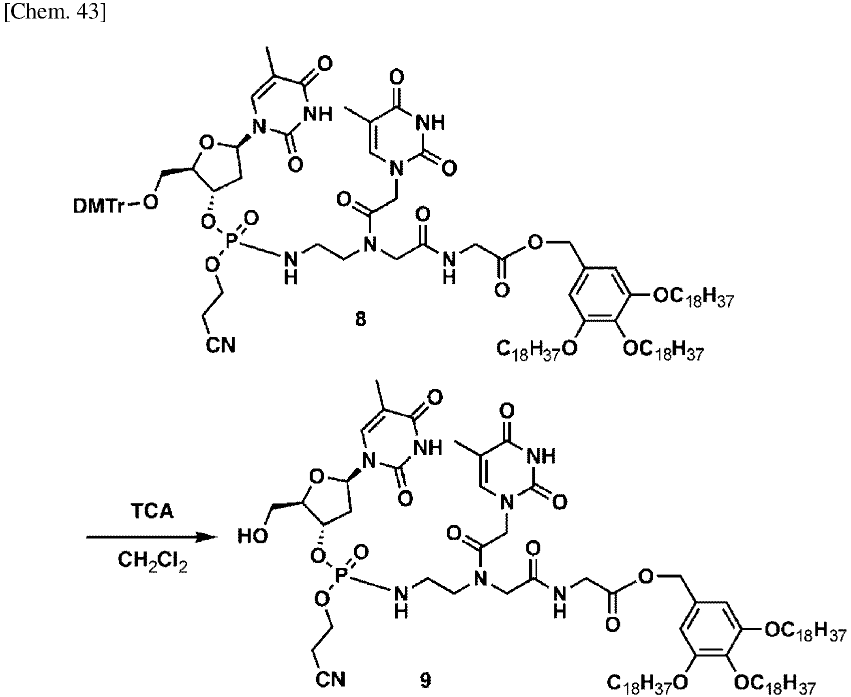

Compound 8 (14.6 mg, 7.70 µmol) was dissolved in dichloromethane (1.00 mL), a 184 mM trichloroacetic acid/dichloromethane solution (2.00 mL) was then added, and the mixture was stirred at room temperature for 15 minutes. Methanol (10.0 mL) was added to the reaction solution, and the obtained suspension was transferred to a centrifuge tube. The supernatant was removed through centrifugation at 3500 rpm for 7 minutes, and the precipitate was recovered. The precipitate was suspended again in methanol (13.0 mL) and centrifuged, and the supernatant was removed. This operation was repeated three times. The precipitate was dried under reduced pressure, whereby Compound 9 was obtained as a white powder (8.60 mg, yield 69.9%).

¹H NMR (400 MHz, CDCl₃) δ 9.21 (m, 2H), 7.51-7.49 (m, 1.5H), 7.04 (m, 2H), 6.56-6.52 (m, 2H), 6.20 (m, 1.5H), 5.30 (s, 1.5H), 5.07-5.01 (m, 3H), 4.70-4.47 (m, 3H), 4.30-4.05 (m, 6H), 3.97-3.85 (m, 8H), 3.49 (m, 4H), 3.38-3.26 (m, 4H), 2.78 (m, 2H), 2.58-2.23 (m, 3H), 2.17 (s, 1H), 2.01 (s, 0.5H), 1.90 (m, 4H), 1.80-1.71 (m, 6H), 1.57 (m, 3H, overlapping with water signal), 1.46 (m, 7H), 1.30-1.25 (m, 73H), 1.11 (s, 2H), 0.89-0.86 (m, 9H) ppm.

³¹P NMR (162 MHz, CDCl₃) δ 9.73, 9.54 ppm.

ESI-TOF-MS C₈₇H₁₄₉N₈NaO₁₆P (M+Na)⁺calcd. m/z 1616.0721, found m/z 1616.0783.

### Example B3 (Separation of lipid-soluble anchor from chimeric molecule)

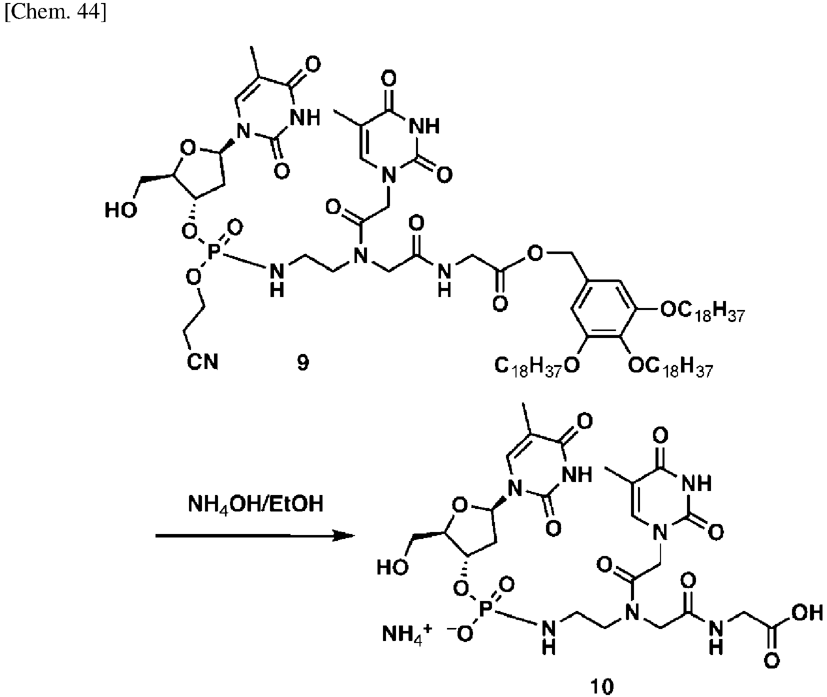

Compound 9 (5.00 mg, 3.14 µmol) was suspended in ethanol (400 µL), and then 28% aqueous ammonia (1.20 mL) was added. The obtained suspension was incubated at 80°C for 10.5 hours, allowed to return to room temperature, and then concentrated. The residue was suspended in methanol (8.00 mL) and transferred to a centrifuge tube. The supernatant was recovered through centrifugation at 3500 rpm for 10 minutes, and the precipitate was removed. The recovered supernatant was concentrated, whereby Compound 10 was obtained as a white solid (318 µg, 0.480 µmol, yield 15.3%).

¹H NMR (400 MHz, D₂O) δ 7.48-7.42 (m, 1H), 7.26-7.14 (m, 1H), 6.08 (dt, J = 13.3, 7.0 Hz, 1H), 4.74 (s, 1H), 4.56-4.45 (m, 1.5H), 4.28-4.16 (m, 1H), 3.97 (s, 2H), 3.81-3.68 (m, 1.5H), 3.68-3.51 (m, 3H), 3.41-3.26 (m, 2H), 3.15 (td, J = 6.5, 1.2 Hz, 2.5H), 3.04-2.88 (m, 2H), 2.75 (td, J = 6.7, 1.1 Hz, 1.5H), 2.35-2.12 (m, 2H), 1.67 (tt, J = 3.1, 1.2 Hz, 6H) ppm. ³¹P NMR (161 MHz, D₂O) δ 8.53, 8.35 ppm. ESI-TOF-MS C₂₃H₃₁N₇O₁₃P (M-H)⁻ calcd. m/z 644.1723, found m/z 644.1963.

### Industrial Applicability

According to the present invention, a method for producing a chimeric molecule by a liquid-phase synthesis method in which a nucleic acid having a neutral or cationic backbone represented by a PNA or a PRNA or a derivative thereof is introduced into a nucleic acid having an anionic backbone represented by an RNA or a DNA or a derivative thereof can be provided.

## Claims

1. A method for producing a chimeric molecule in which at least one first nucleic acid having a neutral or cationic backbone or a derivative thereof and at least one second nucleic acid having an anionic backbone or a derivative thereof are fused, the method comprising:
preparing a second chimeric molecule precursor through introduction of the second nucleic acid or a derivative thereof into a first chimeric molecule precursor having a hydroxyl group via the hydroxyl group of the first chimeric molecule precursor having a hydroxyl group;
deprotecting the second chimeric molecule precursor as necessary; and
introducing the first nucleic acid or a derivative thereof into the second chimeric molecule precursor or the deprotected second chimeric molecule precursor,
wherein
the first chimeric molecule precursor having a hydroxyl group contains a lipid-soluble anchor,
the second nucleic acid or a derivative thereof has an optionally protected amino group,
in the deprotecting of the second chimeric molecule precursor, a protected amino group of the second nucleic acid or a derivative thereof introduced into the second chimeric molecule precursor is deprotected,
in the introducing of the first nucleic acid or a derivative thereof, the first nucleic acid or a derivative thereof is introduced into the second chimeric molecule precursor or the deprotected second chimeric molecule precursor via the amino group of the second nucleic acid or a derivative thereof, and
each of the preparing of the second chimeric molecule precursor, the deprotecting of the second chimeric molecule precursor, and the producing of the chimeric molecule is performed by a liquid-phase synthesis method.

2. The method according to claim 1, wherein the first nucleic acid is a PNA, a PRNA, a PNA/PRNA, or an LNA, and the second nucleic acid is an RNA or a DNA.

3. The method according to claim 2, wherein the first nucleic acid is a PNA, and the second nucleic acid is a DNA.

4. The method according to claim 2, wherein the chimeric molecule includes a portion at which the first nucleic acid or a derivative thereof is bound at a 5'-terminal of the second nucleic acid or a derivative thereof.

5. The method according to claim 1, wherein
the first chimeric molecule precursor is selected from the group consisting of compounds represented by Formulas (1), (2), and (3): 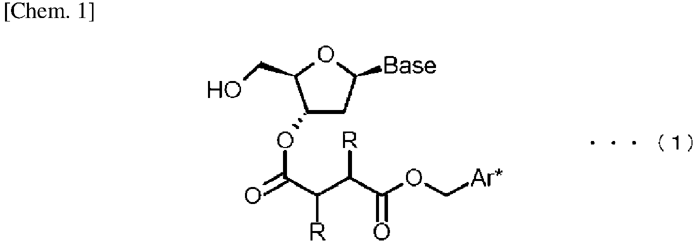 where
Ar* represents an aromatic hydrocarbon ring having 6 to 14 carbon atoms to which an aliphatic hydrocarbon group having 10 to 40 carbon atoms is bound via a single bond or a linker,
R's each independently represent a hydrogen atom or an alkyl group having 1 to 6 carbon atoms, and
Base represents a base moiety, and
deoxyribose may have an optionally protected hydroxyl group at a 2'-position; 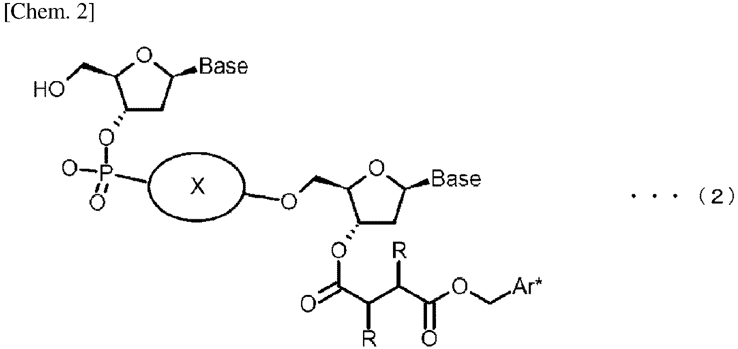 where
Ar* represents an aromatic hydrocarbon ring having 6 to 14 carbon atoms to which an aliphatic hydrocarbon group having 10 to 40 carbon atoms is bound via a single bond or a linker,
R's each independently represent a hydrogen atom or an alkyl group having 1 to 6 carbon atoms,
Base represents a base moiety, each of which may be the same or different, and
X represents a single bond, or represents one or more first nucleic acids and/or second nucleic acids through which an oxygen atom bound to X and a phosphorus atom bound to X are held together, and
deoxyriboses may each independently have an optionally protected hydroxyl group at a 2'- position; and 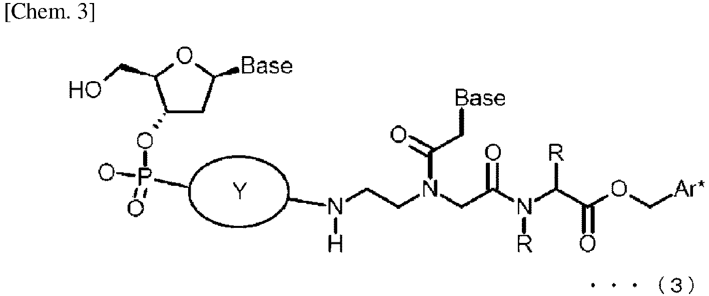 where
Ar* represents an aromatic hydrocarbon ring having 6 to 14 carbon atoms to which an aliphatic hydrocarbon group having 10 to 40 carbon atoms is bound via a single bond or a linker,
R's each independently represent a hydrogen atom or an alkyl group having 1 to 6 carbon atoms;
Base represents a base moiety, each of which may be the same or different, and
Y represents a single bond, or represents one or more first nucleic acids and/or second nucleic acids through which a nitrogen atom bound to Y and a phosphorus atom bound to Y are held together, and
deoxyriboses may each independently have an optionally protected hydroxyl group at a 2'-position.

6. The method according to claim 1, wherein
the second nucleic acid or a derivative thereof is a compound represented by Formula (4): 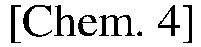 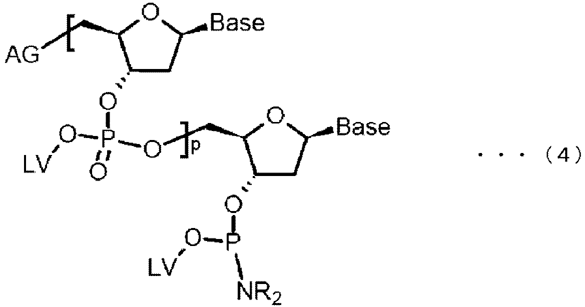 where
p represents an integer of 0 or more,
AG represents an optionally protected amino group,
Base represents a base moiety, each of which may be the same or different,
NR₂ represents a leaving group used in a nucleophilic substitution reaction of an oxygen atom with a phosphorus atom under a weakly acidic condition, and
LV represents a group capable of leaving from an oxygen atom, and
deoxyriboses may each independently have an optionally protected hydroxyl group at a 2'-position.

7. The method according to claim 1, wherein the optionally protected amino group is a protected amino group, and a protective group of the protected amino group is an optionally substituted trityl group.

8. The method according to any one of claims 5 to 7, wherein
the first nucleic acid or a derivative thereof is a compound represented by Formula (5): 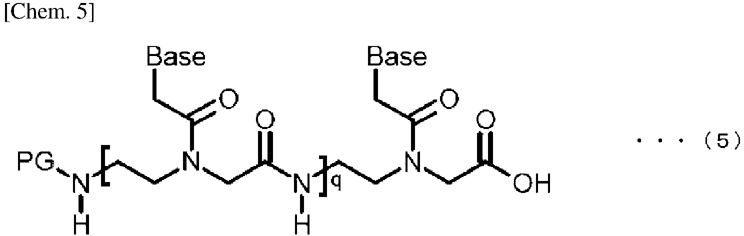 where
q represents an integer of 0 or more,
PG represents a protective group of an adjacent amino group, and
Base represents a base moiety, each of which may be the same or different.

9. A method for producing a chimeric molecule in which at least one first nucleic acid having a neutral or cationic backbone or a derivative thereof and at least one second nucleic acid having an anionic backbone or a derivative thereof are fused,
the method comprising:
introducing the second nucleic acid or a derivative thereof into a third chimeric molecule precursor having an amino group via the amino group of the third chimeric molecule precursor having an amino group,
wherein
the third chimeric molecule precursor having an amino group includes a partial structure derived from the first nucleic acid or a derivative thereof, an amino group derived from the first nucleic acid or a derivative thereof, and a lipid-soluble anchor, and
the introducing of the second nucleic acid or a derivative thereof into the third chimeric molecule precursor having an amino group is performed by a liquid-phase synthesis method.

10. The method according to claim 9, wherein the first nucleic acid is a PNA, a PRNA, a PNA/PRNA, or an LNA, and the second nucleic acid is an RNA or a DNA.

11. The method according to claim 10, wherein the first nucleic acid is a PNA and the second nucleic acid is a DNA.

12. The method according to claim 10, wherein the chimeric molecule has a portion in which the first nucleic acid or a derivative thereof is bound to a 3'-terminal of the second nucleic acid or a derivative thereof.

13. The method according to claim 9, wherein the third chimeric molecule precursor is selected from the group consisting of compounds represented by Formulas (11), (12), and (13): 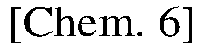 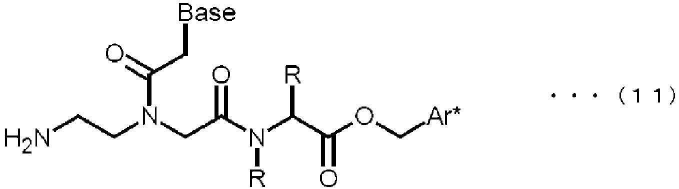 where
Ar* represents an aromatic hydrocarbon ring having 6 to 14 carbon atoms to which an aliphatic hydrocarbon group having 10 to 40 carbon atoms is bound via a single bond or a linker,
R's each independently represent a hydrogen atom or an alkyl group having 1 to 6 carbon atoms, and
Base represents a base moiety; 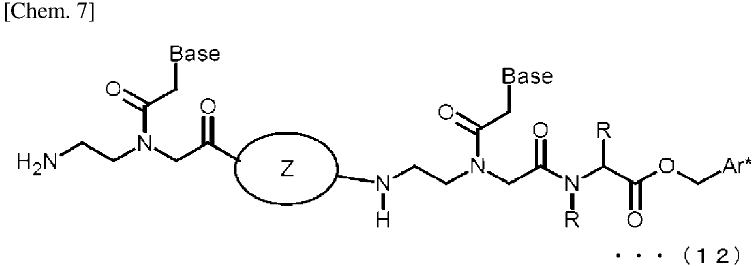 where
Ar* represents an aromatic hydrocarbon ring having 6 to 14 carbon atoms to which an aliphatic hydrocarbon group having 10 to 40 carbon atoms is bound via a single bond or a linker,
R's each independently represent a hydrogen atom or an alkyl group having 1 to 6 carbon atoms,
Base represents a base moiety, and
Z represents a single bond, or represents one or more first nucleic acids and/or second nucleic acids through which a nitrogen atom bound to Z and a carbonyl carbon atom bound to Z are held together, and
deoxyriboses may each independently have an optionally protected hydroxyl group at a 2'-position; and 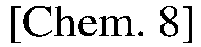 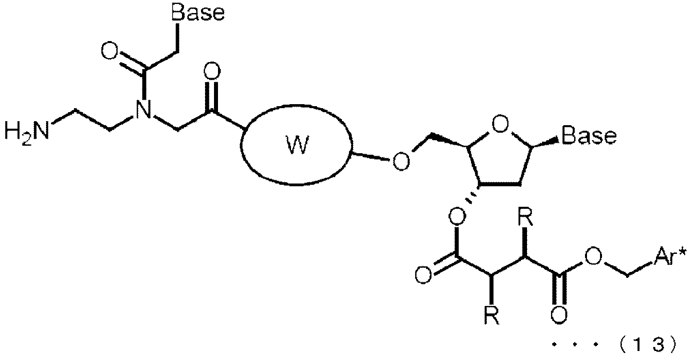 where
Ar* represents an aromatic hydrocarbon ring having 6 to 14 carbon atoms to which an aliphatic hydrocarbon group having 10 to 40 carbon atoms is bound via a single bond or a linker,
R's each independently represent a hydrogen atom or an alkyl group having 1 to 6 carbon atoms,
Base represents a base moiety, and
W represents a single bond, or represents one or more first nucleic acids and/or second nucleic acids through which an oxygen atom bound to W and a carbonyl carbon atom bound to W are held together, and
deoxyriboses may each independently have an optionally protected hydroxyl group at a 2'-position.

14. The method according to claim 9, wherein the second nucleic acid or a derivative thereof is a compound represented by Formula (14): 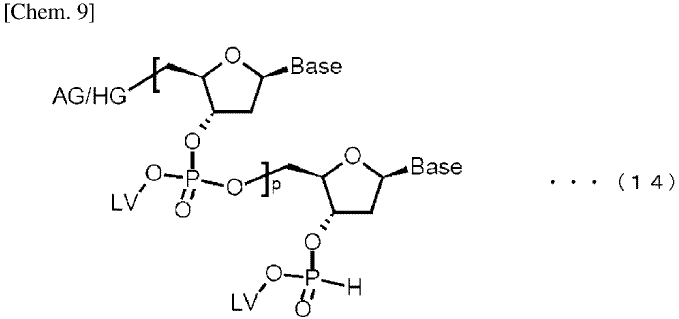 where
p represents an integer of 0 or more,
AG/HG represents an optionally protected amino group or hydroxyl group,
Base represents a base moiety, each of which may be the same or different; and
LV represents a group capable of leaving from an oxygen atom, and
deoxyriboses may each independently have an optionally protected hydroxyl group at a 2'-position.
